# EUROPEAN PATENT APPLICATION

(11) **EP 2 666 479 A2**
(43) Date of publication of application: **27.11.2013**
(21) Application number: 13172516.0
(22) Date of filing: 10.04.2007
(51) Int. Cl.: A61K 39/395, C07K 16/24

(54) **Uses and compositions for treatment of juvenile rheumatoid arthritis**

(30) Priority: 10.04.2006 US 790909 P; 30.05.2006 US 809770 P; 20.06.2006 US 815489 P; 10.11.2006 US 858376 P
(62) Divisional of application: 07755184.4
(71) Applicant: Abbott Biotechnology Ltd, HM 11 Hamilton (BM)
(72) Inventor: Giannini, Edward, H., Cincinnati, OH Ohio 45220 (US); Medich, John R., East Hanover, NJ New Jersey 07936 (US); Martini, Alberto, Piacenza (IT); Lovell, Daniel J., Cincinnati, OH Ohio 45220 (US); Ruperto, Nicolino, 16035 Rapallo (IT)
(74) Representative: Modiano, Micaela Nadia

(57) **Abstract**

The invention provides methods, uses and compositions for the treatment of juvenile rheumatoid arthritis (JRA). The invention describes methods and uses for treating JRA, wherein a TNFa inhibitor, such as a human TNFa antibody, or antigenbinding portion thereof, is used to prevent flare-ups associated with JRA. Also described are methods for determining the efficacy of a TNFa inhibitor for treatment of JRA in a subject.

SEQUENCE LISTING

<110> Abbott Biotechnology Ltd.

<120> USES AND COMPOSITIONS FOR TREATMENT OF JUVENILE
      RHEUMATOID ARTHRITIS

<130> 117813/28521

<140> EP07755184.4
<141> 2008-11-10

<150> 60/790909
<151> 2006-04-10

<150> 60/809770
<151> 2006-05-30

<150> 60/815489
<151> 2006-06-30

<150> 60/858376
<151> 2006-11-10

<160> 37

<170> FastSEQ for Windows Version 4.0

<210> 1
<211> 107
<212> PRT
<213> Artificial Sequence

<220>
<223> adalimumab light chain variable region

<400> 1

<210> 2
<211> 121
<212> PRT
<213> Artificial Sequence

<220>
<223> adalimumab heavy chain variable region

<400> 2

<210> 3
<211> 9
<212> PRT
<213> Artificial Sequence

<220>
<223> adalimumab light chain variable region CDR3

<221> VARIANT
<222> 9
<223> Xaa = Thr or Ala

<400> 3
Gln Arg Tyr Asn Arg Ala Pro Tyr Xaa
 1               5

<210> 4
<211> 12
<212> PRT
<213> Artificial Sequence

<220>
<223> adalimumab heavy chain variable region CDR3

<221> VARIANT
<222> 12
<223> Xaa = Tyr or Asn

<400> 4
Val Ser Tyr Leu Ser Thr Ala Ser Ser Leu Asp Xaa
 1               5                   10

<210> 5
<211> 7
<212> PRT
<213> Artificial Sequence

<220>
<223> adalimumab light chain variable region CDR2

<400> 5
Ala Ala Ser Thr Leu Gln Ser
 1               5

<210> 6
<211> 17
<212> PRT

<213> Artificial Sequence

<220>
<223> adalimumab heavy chain variable region CDR2

<400> 6
Ala Ile Thr Trp Asn Ser Gly His Ile Asp Tyr Ala Asp Ser Val Glu
1               5                   10                  15
Gly

<210> 7
<211> 11
<212> PRT
<213> Artificial Sequence

<220>
<223> adalimumab light chain variable region CDR1

<400> 7
Arg Ala Ser Gln Gly Ile Arg Asn Tyr Leu Ala
1               5                   10

<210> 8
<211> 5
<212> PRT
<213> Artificial Sequence

<220>
<223> adalimumab heavy chain variable region CDR1

<400> 8
Asp Tyr Ala Met His
1               5

<210> 9
<211> 107
<212> PRT
<213> Artificial Sequence

<220>
<223> 2SD4 light chain variable region

<400> 9
Asp Ile Gln Met Thr Gln Ser Pro Ser Ser Leu Ser Ala Ser Ile Gly
1               5                   10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Tyr
                85                  90                  95
Ala Phe Gly Gln Gly Thr Lys Val Glu Ile Lys
            100                 105

<210> 10
<211> 121
<212> PRT

<213> Artificial Sequence

<220>
<223> 2SD4 heavy chain variable region

<400> 10
Gln Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Asp Trp Val
        35                  40                  45
Ser Ala Ile Thr Trp Asn Ser Gly His Ile Asp Tyr Ala Asp Ser Val
    50                  55                  60
Glu Gly Arg Phe Ala Val Ser Arg Asp Asn Ala Lys Asn Ala Leu Tyr
65                  70                  75                  80
Leu Gln Met Asn Ser Leu Arg Pro Glu Asp Thr Ala Val Tyr Tyr Cys
                85                  90                  95
Thr Lys Ala Ser Tyr Leu Ser Thr Ser Ser Ser Leu Asp Asn Trp Gly
            100                 105                 110
Gln Gly Thr Leu Val Thr Val Ser Ser
        115                 120

<210> 11
<211> 9
<212> PRT
<213> Artificial Sequence

<220>
<223> 2SD4 light chain variable region CDR3

<400> 11
Gln Lys Tyr Asn Ser Ala Pro Tyr Ala
1               5

<210> 12
<211> 9
<212> PRT
<213> Artificial Sequence

<220>
<223> EP B12  light chain variable region CDR3

<400> 12
Gln Lys Tyr Asn Arg Ala Pro Tyr Ala
1               5

<210> 13
<211> 9
<212> PRT
<213> Artificial Sequence

<220>
<223> VL10E4 light chain variable region CDR3

<400> 13
Gln Lys Tyr Gln Arg Ala Pro Tyr Thr
1               5

<210> 14
<211> 9
<212> PRT
<213> Artificial Sequence

<220>
<223> VL100A9 light chain variable region CDR3

<400> 14
Gln Lys Tyr Ser Ser Ala Pro Tyr Thr
1               5

<210> 15
<211> 9
<212> PRT
<213> Artificial Sequence

<220>
<223> VLL100D2 light chain variable region CDR3

<400> 15
Gln Lys Tyr Asn Ser Ala Pro Tyr Thr
1               5

<210> 16
<211> 9
<212> PRT
<213> Artificial Sequence

<220>
<223> VLL0F4 light chain variable region CDR3

<400> 16
Gln Lys Tyr Asn Arg Ala Pro Tyr Thr
1               5

<210> 17
<211> 9
<212> PRT
<213> Artificial Sequence

<220>
<223> LOE5 light chain variable region CDR3

<400> 17
Gln Lys Tyr Asn Ser Ala Pro Tyr Tyr
1               5

<210> 18
<211> 9
<212> PRT
<213> Artificial Sequence

<220>
<223> VLLOG7 light chain variable region CDR3

<400> 18
Gln Lys Tyr Asn Ser Ala Pro Tyr Asn
1               5

<210> 19
<211> 9
<212> PRT
<213> Artificial Sequence

<220>
<223> VLLOG9 light chain variable region CDR3

<400> 19
Gln Lys Tyr Thr Ser Ala Pro Tyr Thr
1               5

<210> 20
<211> 9
<212> PRT
<213> Artificial Sequence

<220>
<223> VLLOH1 light chain variable region CDR3

<400> 20
Gln Lys Tyr Asn Arg Ala Pro Tyr Asn
1               5

<210> 21
<211> 9
<212> PRT
<213> Artificial Sequence

<220>
<223> VLLOH10 light chain variable region CDR3

<400> 21
Gln Lys Tyr Asn Ser Ala Ala Tyr Ser
1               5

<210> 22
<211> 9
<212> PRT
<213> Artificial Sequence

<220>
<223> VL1B7 light chain variable region CDR3

<400> 22
Gln Gln Tyr Asn Ser Ala Pro Asp Thr
1               5

<210> 23
<211> 9
<212> PRT
<213> Artificial Sequence

<220>
<223> VL1C1 light chain variable region CDR3

<400> 23
Gln Lys Tyr Asn Ser Asp Pro Tyr Thr
1               5

<210> 24

<211> 9
<212> PRT
<213> Artificial Sequence

<220>

<223> VL0.1F4 light chain variable region CDR3

<400> 24
Gln Lys Tyr Ile Ser Ala Pro Tyr Thr
1               5

<210> 25
<211> 9
<212> PRT
<213> Artificial Sequence

<220>
<223> VL0.1H8 light chain variable region CDR3

<400> 25
Gln Lys Tyr Asn Arg Pro Pro Tyr Thr
1               5

<210> 26
<211> 9
<212> PRT
<213> Artificial Sequence

<220>
<223> LOE7.A light chain variable region CDR3


<400> 26
Gln Arg Tyr Asn Arg Ala Pro Tyr Ala
1               5

<210> 27
<211> 12
<212> PRT
<213> Artificial Sequence

<220>
<223> 2SD4 heavy chain variable region CDR3

<400> 27
Ala Ser Tyr Leu Ser Thr Ser Ser Ser Leu Asp Asn
1               5                   10

<210> 28
<211> 12
<212> PRT
<213> Artificial Sequence

<220>
<223> VH1B11 heavy chain variable region CDR3

<400> 28
Ala Ser Tyr Leu Ser Thr Ser Ser Ser Leu Asp Lys
1               5                   10

<210> 29
<211> 12
<212> PRT
<213> Artificial Sequence

<220>
<223> VH1D8 heavy chain variable region CDR3

<400> 29
Ala Ser Tyr Leu Ser Thr Ser Ser Ser Leu Asp Tyr
1               5                   10

<210> 30
<211> 12
<212> PRT
<213> Artificial Sequence

<220>
<223> VH1A11 heavy chain variable region CDR3

<400> 30
Ala Ser Tyr Leu Ser Thr Ser Ser Ser Leu Asp Asp
1               5                   10

<210> 31
<211> 12
<212> PRT
<213> Artificial Sequence

<220>
<223> VH1B12 heavy chain variable region CDR3

<400> 31
Ala Ser Tyr Leu Ser Thr Ser Phe Ser Leu Asp Tyr
1               5                   10

<210> 32
<211> 12
<212> PRT
<213> Artificial Sequence

<220>
<223> VH1E4 heavy chain variable region CDR3

<400> 32
Ala Ser Tyr Leu Ser Thr Ser Ser Ser Leu His Tyr
1               5                   10

<210> 33
<211> 12
<212> PRT
<213> Artificial Sequence

<220>
<223> VH1F6 heavy chain variable region CDR3

<400> 33
Ala Ser Phe Leu Ser Thr Ser Ser Ser Leu Glu Tyr
1               5                   10

<210> 34
<211> 12
<212> PRT
<213> Artificial Sequence

<220>
<223> 3C-H2 heavy chain variable region CDR3

<400> 34

Ala Ser Tyr Leu Ser Thr Ala Ser Ser Leu Glu Tyr
 1               5                   10

<210> 35
<211> 12
<212> PRT
<213> Artificial Sequence

<220>
<223> VH1-D2.N heavy chain variable region CDR3

<400> 35
Val Ser Tyr Leu Ser Thr Ala Ser Ser Leu Asp Asn
 1               5                   10

<210> 36
<211> 321
<212> DNA
<213> Artificial Sequence

<220>
<223> adalimumab light chain variable region

<400> 36
gacatccaga tgacccagtc tccatcctcc ctgtctgcat ctgtagggga cagagtcacc 60
atcacttgtc gggcaagtca gggcatcaga aattacttag cctggtatca gcaaaaacca 120
gggaaagccc ctaagctcct gatctatgct gcatccactt tgcaatcagg ggtcccatct 180
cggttcagtg gcagtggatc tgggacagat ttcactctca ccatcagcag cctacagcct 240
gaagatgttg caacttatta ctgtcaaagg tataaccgtg caccgtatac ttttggccag 300
gggaccaagg tggaaatcaa a                                           321

<210> 37
<211> 363
<212> DNA
<213> Artificial Sequence

<220>
<223> adalimumab heavy chain variable region

<400> 37
gaggtgcagc tggtggagtc tgggggaggc ttggtacagc ccggcaggtc cctgagactc 60
tcctgtgcgg cctctggatt cacctttgat gattatgcca tgcactgggt ccggcaagct 120
ccagggaagg gcctggaatg ggtctcagct atcacttgga atagtggtca catagactat 180
gcggactctg tggagggccg attcaccatc tccagagaca acgccaagaa ctccctgtat 240
ctgcaaatga acagtctgag agctgaggat acggccgtat attactgtgc gaaagtctcg 300
taccttagca ccgcgtcctc ccttgactat tggggccaag gtaccctggt caccgtctcg 360
agt                                                               363

## Description

### RELATED APPLICATIONS

This application claims the benefit of priority to U.S. provisional patent application number 60/790909 filed on April 10, 2006; U.S. provisional patent application number 60/809770 filed on May 30, 2006; U.S. provisional patent application number 60/815489 filed on June 20, 2006; and U.S. provisional patent application number 60/858376 filed on November 10, 2006. The contents of all the above-mentioned priority applications are hereby incorporated by reference in their entirety.

### BACKGROUND

Juvenile rheumatoid arthritis (JRA), also referred to as juvenile chronic polyarthritis, Still's disease, and juvenile idiopathic arthritis (JIA) is the most common type of arthritis affecting children. Rheumatoid arthritis is a chronic disease that can affect joints in any part of the body. JRA·is an arthritis that causes joint inflammation and stiffness for more than 6 weeks in a child less than 16 years of age.

In this disease, the immune system mistakenly targets the synovium. The synovium responds by making excess synovial fluid, which leads to swelling, pain and stiffness. The inflammation can then spread to the surrounding tissues, eventually damaging cartilage and bone. Other areas of the body, including the eyes, kidneys, lungs and heart, also may be affected by the inflammation. Without treatment, JRA can interfere with a child's normal growth and development.

### SUMMARY OF THE INVENTION

There remains a need for an effective and safe treatment option for patients suffering from juvenile rheumatoid arthritis (JRA). The instant invention provides improved methods and compositions for treating JRA, including flare-ups associated with JRA. The invention further provides a means by which the efficacy of a TNFα inhibitor for the treatment of JRA can be determined. Kits and labels which provide information pertaining to the methods, uses, and compositions of the invention are also described herein. Each of the examples described herein describes methods which can be used to determine whether a TNFα inhibitor is effective for treating the given disorder.

The invention describes a method of preventing flare ups associated with juvenile arthritis (JRA) comprising administering adalimumab to a patient having JRA, such that flare ups are prevented. In one embodiment, the flare up is prevented from occurring for at least about 32 weeks. In another embodiment, the flare up is prevented from occurring for at least about 48 weeks.

The invention provides an article of manufacture comprising a packaging material; a TNFα inhibitor; and a label or package insert contained within the packaging material indicating that in studies of the TNFα inhibitor for the treatment of juvenile rheumatoid arthritis (JRA) the most common adverse events (AEs) were infections. In one embodiment, the infections include mild upper respiratory infections.

The invention describes a method of preventing flare ups associated with juvenile arthritis (JRA) comprising administering adalimumab to a patient having JRA, such that flare ups are prevented. In one embodiment, the flare up is prevented from occurring for at least about 32 week. In another embodiment, the flare up is prevented from occurring for at least about 48 weeks.

The invention describes a method of preventing flare ups associated with juvenile arthritis (JRA) comprising administering adalimumab to a patient having JRA, such that flare ups are prevented. In one embodiment, the flare up is prevented from occurring for at least about 32 weeks. In another embodiment, the flare up is prevented from occurring for at least about 48 weeks.

The invention provides a method of preventing a flare-up associated with juvenile rheumatoid arthritis (JRA) in a subject comprising administering a TNFα antibody, or antigen-binding portion thereof, to a subject, such that a flare up is prevented.

The invention provides use of a TNFα antibody, or antigen-binding portion thereof, in the manufacture of a medicament for preventing a flare-up associated with juvenile rheumatoid arthritis (JRA).

The invention provides a method for determining the efficacy of a TNFα antibody, or antigen-binding portion thereof, for the prevention of a flare-up associated with JRA in a subject, comprising determining a median time to flare of a patient population having JRA and who was administered the TNFα antibody, or antigen-binding portion thereof, wherein a median time to flare of at least about 14 weeks indicates that the TNFα antibody, or antigen-binding portion thereof, is an effective TNFα antibody, or antigen-binding portion thereof, for the prevention of flare associated with JRA. In one embodiment, the method further comprises administering the effective TNFα antibody, or antigen-binding portion thereof, to a subject to prevent a flare-up associated with JRA.

The invention provides a method of preventing a flare up in a subject having JRA comprising administering an effective TNFα antibody, or antigen-binding portion thereof, to a subject such that flare-up is prevented, wherein the effective TNFα antibody, or antigen-binding portion thereof, was previously identified as preventing a flare-up for at least about 14 weeks in a patient population having JRA.

The invention provides a method for determining the efficacy of a TNFα antibody, or antigen-binding portion thereof, for the prevention of a flare-up associated with JRA in a subject, comprising determining a flare-up occurrence rate of a patient population having JRA and who was administered the TNFα antibody, or antigen-binding portion thereof, wherein a flare-up occurrence rate of about 43% or less indicates that the TNFα antibody, or antigen-binding portion thereof, is an effective TNFα antibody, or antigen-binding portion thereof, for the prevention of the flare-up associated with JRA. In one embodiment, the method further comprises administering the effective TNFα antibody, or antigen-binding portion thereof, to a subject for the prevention of a flare-up associated with JRA.

*The invention provides a method of preventing a flare up in a subject having* JRA comprising administering an effective TNFα antibody, or antigen-binding portion thereof, to a subject such that the flare-up is prevented, wherein the effective TNFα antibody, or antigen-binding portion thereof, was previously identified as preventing a flare-up in about 43% or less of a patient population having JRA.

In one embodiment, a flare-up occurrence rate of about 40% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 37% or less indicates that the TNFα antibody, or antigen-binding portion thereof, is an effective TNFα antibody, or antigen-binding portion thereof, for the prevention of the flare-up associated with JRA.

In one embodiment, the flare-up is prevented for at least 20 weeks. In another embodiment, the flare-up is prevented for at least 14 weeks. In one embodiment, the TNFα antibody, or antigen-binding portion thereof, is administered at a dose of 24 mg/M² BSA. In one embodiment, the TNFα antibody, or antigen-binding portion thereof, is administered subcutaneously. In one embodiment, the TNFα antibody, or antigen-binding portion thereof, is administered on a biweekly dosing regimen.

In one embodiment, the TNFα antibody, or antigen-binding portion thereof, is administered with methotrexate. In another embodiment, the TNFα antibody, or antigen-binding portion thereof, is administered without methotrexate.

The invention provides a method for determining the efficacy of a TNFα inhibitor for the treatment of juvenile rheumatoid arthritis (JRA) in a subject comprising determining a Pediatric (Ped) ACR90 response of a patient population having JRA and who was administered the TNFα inhibitor, wherein a Ped ACR90 response achieved in at least about 15% of the patient population indicates that the TNFα inhibitor is an effective TNFα inhibitor for treating JRA. In one embodiment, the method further comprises administering the effective TNFα inhibitor to a subject to treat JRA. In one embodiment, the TNFα inhibitor is administered either with or without methotrexate. In one embodiment, a Ped ACR90 response achieved in at least about 20% of the patient population indicates that the TNFα inhibitor is an effective TNFα inhibitor for treating JRA. In another embodiment, a Ped ACR90 response achieved in at least about 30% of the patient population indicates that the TNFα inhibitor is an effective TNFα inhibitor for treating JRA. In yet another embodiment, a Ped ACR90 response achieved in at least about 40% of the patient population indicates that the TNFα inhibitor is an effective TNFα inhibitor for treating JRA.

The invention provides a method for treating JRA in a subject comprising administering an effective TNFα inhibitor to the subject such that JRA is treated, wherein the effective TNFα inhibitor was previously identified as achieving a PedACR90 response in at least about 15 % of a patient population having JRA. In one embodiment, the method further comprises administering methotrexate to the subject. In one embodiment, the effective TNFα inhibitor was previously identified as achieving a Ped ACR90 response in at least about 20% of a patient population having JRA. In another embodiment, the effective TNFα inhibitor was previously identified as achieving a Ped ACR90 response in at least about 30% of a patient population having JRA. In yet another embodiment, the effective TNFα inhibitor was previously identified as achieving a Ped ACR90 response in at least about 40% of a patient population having JRA. In another embodiment, the effective TNFα inhibitor was previously identified as achieving a Ped ACR90 response in at least about 42% of a patient population having JRA.

The invention provides an article of manufacture comprising a packaging material; a TNFα antibody, or antigen-binding portion thereof; and a label or package insert indicating that in studies of the TNFα antibody, or antigen-binding portion thereof, for the treatment of juvenile rheumatoid arthritis (JRA) the most common adverse events (AEs) were infections.

The invention provides an article of manufacture comprising a packaging material; a TNFα antibody, or antigen-binding portion thereof; and a label or package insert indicating that in studies of the TNFα antibody, or antigen-binding portion thereof, the TNFα antibody was shown to prevent flare-ups in patients having JRA.

In one embodiment, the JRA is polyarticular JRA.

In one embodiment, the TNFα antibody, or antigen-binding portion thereof, is selected from the group consisting of adalimumab, infliximab, and golimumab.

In one embodiment, the TNFα inhibitor is administered weekly to the patient population. In one embodiment, the TNFα inhibitor is administered biweekly to the patient population.

In one embodiment, the TNFα inhibitor is administered in a biweekly dosing regimen. In one embodiment, the TNFα inhibitor is administered weekly. In another embodiment, the TNFα inhibitor is administered every other week.

In one embodiment, the TNFα inhibitor is administered as a monotherapy.

In another embodiment, the TNFα inhibitor is administered with an additional therapeutic agent. In one embodiinent, the TNFα inhibitor is administered with methotrexate. In one embodiment, the patient or patient population is administered methotrexate in combination with the TNFα inhibitor.

In one embodiment, the TNFα inhibitor is selected from the group consisting of a TNFα antibody, or an antigen-binding portion thereof, a TNF fusion protein, or a recombinant TNF binding protein.

In one embodiment, the TNF fusion protein is etanercept.

In one embodiment, the TNFα antibody, or antigen-binding portion thereof, is infliximab or golimumab.

In one embodiment, the TNFα antibody, or antigen-binding portion thereof, is selected from the group consisting of a chimeric antibody, a humanized antibody, and a multivalent antibody. In one embodiment, the TNFα antibody, or antigen-binding portion thereof, is a human antibody.

In one embodiment, the TNFα antibody, or antigen-binding portion thereof, is an isolated human antibody that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a *standard in vitro* L929 assay with an IC₅₀ of 1 x 10⁻⁷ M or less.

In one embodiment, the TNFα antibody is an isolated human antibody, or antigen-binding portion thereof, with the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID *NO*: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In one embodiment, the TNFα antibody is an isolated human antibody, or an antigen binding portion thereof, with a light chain variable region (LCVR) comprising the amino acid sequence of SEQ ID NO: 1 and a heavy chain variable region (HCVR) comprising the amino acid sequence of SEQ ID NO: 2

In one embodiment, the human TNFα antibody, or antigen-binding portion thereof, is adalimumab.

In one embodiment, the TNFα antibody, or antigen-binding portion thereof, is a 24 mg Ada/M² BSA dose.

In one embodiment, the TNFα antibody, or antigen-binding portion thereof, is administered subcutaneously. In one embodiment, the TNFα antibody, or antigen-binding portion thereof, is administered in a biweekly dosing regimen.

### BREIF DESCRIPTION OF THE FIGURES

*Figure 1*graphically depicts the design of the study in the Examples, including the open label extension.
*Figure 2* shows the patient disposition.
*Figure 3* graphically depicts KM estimate of time to flare for patients without MTX during double-blind phase
*Figure 4* graphically depicts KM estimate of time to flare for patients with MTX during double-blind phase.
*Figure 5* graphically depicts the open-label ACR Pedi 30 responses by DB treatment assignment.
*Figure 6* graphically depicts the open-label ACR Pedi 70 responses by DB treatment assignment.
*Figure 7* graphically depicts PedACR Responses during the open-label extension phase.

### DETAILED DESCRIPTION OF THE INVENTION

### I. Definitions

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovatently bound 17 kD molecules. The structure of hTNFα is described further in, for example, Pennica, D., et al. (1984) Nature 312:724-729; Davis, J,M., et al. (1987) Biochemistry 26:1322-1326; and Jones, E.Y., et al. (1989) Nature 335:225-228. The term human TNFα is intended to include recombinant human TNFα (rhTNFα), which can be prepared by standard recombinant expression methods or purchased commercially (R & D Systems, Catalog No. 210-TA, Minneapolis, MN). TNFα is also referred to as TNF,

The term "TNFα inhibitor" includes agents which interfere with TNFα activity. The term also includes each of the anti-TNFα human antibodies and antibody portions described herein as well as those described in U.S. Patent Nos. 6,090,382; 6,258,562; 6,509,015, and in U.S. Patent Application Serial Nos. 09/801185 and 10/302356, In one embodiment, the TNFα inhibitor used in the invention is an anti-TNFα antibody, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA®^{®} Abbott Laboratories, a human anti-TNF mAb, described in US 6,090,382 as D2E7). Additional TNF antibodies which may be used in the invention are described in U.S. Patent Nos. 6,593,458; 6,498,237; 6,451,983; and 6,448,380, each of which is incorporated by reference herein. In another embodiment, the TNFα inhibitor is a TNF fusion protein, e.g., etanercept (Enbrel^{®}, Amgen; described in WO 91/03553 and WO 09/406476, incorporated by reference herein). In another embodiment, the TNFα inhibitor is a recombinant TNF binding protein (r-TBP-I) (Serono).

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The antibodies of the invention are described in further detail in U.S. Patent Nos. 6,090,382; 6,258,562; and 6,509,015, each of which is incorporated herein by reference in its entirety.

The term "antigen-binding portion" or "antigen-binding fragment" of an antibody (or simply "antibody portion"), as used herein, refers to one or more fragments of an antibody that retain the ability to specifically bind to an antigen (*e.g*., hTNFα). It has been shown that the antigen-binding function of an antibody can be performed by fragments of a full-length antibody. Binding fragments include Fab, Fab', F(ab')₂, Fabc, Fv, single chains, and single-chain antibodies. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 341:544-546 ), which consists of a VH domain; and (vi) an isolated complementarity determining region (CDR2). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g*., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.*, Holliger et al. (1993) Proc. Natl. Acad. Sci. USA 90:6444-6448; Poljak et al. (1994) Structure 2:1121-1123). The antibody portions of the invention are described in further detail in U.S. Patent Nos. 6,090,382, 6,258,562, 6,509,015, each of which is incorporated herein by reference in its entirety.

Still further, an antibody or antigen-binding portion thereof may be part of a larger immunoadhesion molecules, formed by covalent or noncovalent association of the antibody or antibody portion with one or more other proteins or peptides. Examples of such immunoadhesion molecules include use of the streptavidin core region to make a tetrameric scFv molecule (Kipriyanov, S.M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S.M., et al. (1994) Mol. Immunol. 31:1.047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

A "conservative amino acid substitution", as used herein, is one in which one amino acid residue is replaced with another amino acid residue having a similar side chain. Families of amino acid residues having similar side chains have been defined in the art, including basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine).

"Chimeric antibodies" refers to antibodies wherein one portion of each of the amino acid sequences of heavy and light chains is homologous to corresponding sequences in antibodies derived from a particular species or belonging to a particular class, while the remaining segment of the chains is homologous to corresponding sequences from another species. In one embodiment, the invention features a chimeric antibody or antigen-binding fragment, in which the variable regions of both light and heavy chains mimics the variable regions of antibodies derived from one species of mammals, while the constant portions are homologous to the sequences in antibodies derived from another species. In a preferred embodiment of the invention, chimeric antibodies are made by grafting CDRs from a mouse antibody onto the framework regions of a human antibody.

"Humanized antibodies" refer to antibodies which comprise at least one chain comprising variable region framework residues substantially from a human antibody chain (referred to as the acceptor immunoglobulin or antibody) and at least one complementarity determining region (CDR) substantially from a non-human-antibody (e.g., mouse). In addition to the grafting of the CDRs, humanized antibodies typically undergo further alterations in order to improve affinity and/or immmunogenicity.

The term "multivalent antibody" refers to an antibody comprising more than one antigen recognition site. For example, a "bivalent" antibody has two antigen recognition sites, whereas a "tetravalent" antibody has four antigen recognition sites. The terms "monospecific", "bispecific", "trispecifie", "tetraspecific", etc. refer to the number of different antigen recognition site specificities (as opposed to the number of antigen recognition sites) present in a multivalent antibody. For example, a "monospecific" antibody's antigen recognition sites all bind the same epitope. A "bispecific" or "dual specific" antibody has at least one antigen recognition site that binds a first epitope and at least one antigen recognition site that binds a second epitope that is different from the first epitope. A "multivalent monospecific" antibody has multiple antigen recognition sites that all bind the same epitope. A "multivalent bispecific" antibody has multiple antigen recognition sites, some number of which bind a first epitope and some number of which bind a second epitope that is different from the first epitope

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo),* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (*e.g.*, a mouse) that is transgenic for human immunoglobulin genes (see *e.g.*, Taylor et al. (1992) Nucl. Acids Res. 20:6287) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

Such chimeric, humanized, human, and dual specific antibodies can be produced by recombinant DNA techniques known in the art, for example using methods described in PCT International Application No. PCT/US86/02269; European Patent Application No. 184,187; European Patent Application No. 171,496; European Patent Application No. 173,494; PCT International Publication No. WO 86/01533; U.S. Pat. No. 4,816,567; European Patent Application No. 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Cancer Res. 47:999-1005; Wood et al. (1985) Nature 314:446-449; Shaw et al. (1988) J. Natl. cancer Inst. 80:1553-1559); Morrison (1985) Science 229:1202-1207; Oi et al. (1986) BioTechniques 4:214; U.S. Pat. No. 5,225,539; Jones et al. (1986) Nature 321:552-525; Verhoeyan et al. (1988) Science 239:1534; and Beidler et al. (1988) J. Immunol. 141:4053-4060, Queen et al., Proc. Natl. Acad. Sci. USA 86:10029-10033 (1989), US 5,530,101, US 5,585,089, US 5,693,761, US 5,693,762, Selick et al., WO 90/07861, and Winter, US 5,225,539.

An "isolated antibody", as used herein, is intended to refer to an antibody that is substantially free of other antibodies having different antigenic specificities (*e*.*g*., an isolated antibody that specifically binds hTNFα is substantially free of antibodies that specifioally_bind antigens other than hTNFα). An isolated antibody that specifically binds hTNFα may, however, have cross-reactivity to other antigens, such as TNFα molecules from other species. Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or *in vivo),* hTNFα-induced *cellular activation and hTNFα binding to hTNFα receptors. These indicators* of hTNFα biological activity can be assessed by one or more of several *standard in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 of U.S. Patent 6,258,562 and Jönsson et al. (1993) Ann. Biol. Clin. 51:19; Jönsson et al. (1991) Biotechniques 11:620-627; Johnsson et al.(1995) J. Mol. Recognit. 8:125; and Johnnson et al. (1991) Anal. Biochem. 198:268.

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

The term "IC₅₀" as used herein, is intended to refer to the concentration of the inhibitor required to inhibit the biological endpoint of interest, *e.g.*, neutralize cytotoxicity activity.

The term "dose," as used herein, refers to an amount of TNFα inhibitor which is administered to a subject.

The term "dosing", as used herein, refers to the administration of a substance (*e.g.*, an anti-TNFα antibody) to achieve a therapeutic objective (*e*.*g*., treatment of juvenile rheumatoid arthritis).

A "dosing regimen" describes a treatment schedule for a TNFα inhibitor, *e*.*g*., a treatment schedule over a prolonged period of time and/or throughout the course of treatment, *e.g.* administering a first dose of a TNFα inhibitor at week 0 followed by a second dose of a TNFα, inhibitor on a biweekly dosing regimen.

The terms "biweekly dosing regimen", "biweekly dosing", and "biweekly administration", as used herein, refer to the time course of administering a substance *(e.g.,* an anti-TNFα antibody) to a subject to achieve a therapeutic objective, *e.g,* throughout the course of treatment. The biweekly dosing regimen is not intended to include a weekly dosing regimen. Preferably, the substance is administered every 9-19 days, more preferably, every 11-17 days, even more preferably, every 13-15 days, and most preferably, every 14 days. In one embodiment, the biweekly dosing regimen is initiated in a subject at week 0 of treatment. In another embodiment, a maintenance dose is administered on a *biweekly dosing regimen. In one embodiment, biweekly* dosing includes a dosing regimen wherein doses of a TNFα inhibitor are administered to a subject every other week beginning at week 0. In one embodiment, biweekly dosing includes a dosing regimen where doses of a TNFα inhibitor are administered to a subject every other week consecutively for a given time period, e.g., 4 weeks, 8 weeks, 16, weeks, 24 weeks, 26 weeks, 32 weeks, 36 weeks, 42 weeks, 48 weeks, 52 weeks, 56 weeks, etc. Biweekly dosing methods are also described in US 20030235585, incorporated by reference herein.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present invention, therefore, includes methods of combination therapeutic treatment and combination pharmaceutical composition.

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (*e.g.*, human).

The term "combination therapy", as used herein, refers to the administration of two or more therapeutic substances, *e.g.*, an anti-TNFα antibody and another drug. The other drug(s) may be administered concomitant with, prior to, or following the administration of an anti-TNFα antibody.

The term "treatment," as used within the context of the present invention, is meant to include therapeutic treatment, as well as prophylactic or suppressive measures, for the treatment of juvenile rheumatoid arthritis. For example, the term treatment may include administration of a TNFα inhibitor prior to or following the onset of juvenile rheumatoid arthritis thereby preventing or removing signs of the disease or disorder. As another example, administration of a TNFα inhibitor after clinical manifestation of *juvenile rheumatoid arthritis to combat the symptoms and*/*or complications and* disorders associated with juvenile rheumatoid arthritis comprises "treatment" of the disease. Further, administration of the agent after onset and after clinical symptoms and/or complications have developed where administration affects clinical parameters of the disease or disorder and perhaps amelioration of the disease, comprises "treatment" of the juvenile rheumatoid arthritis. In one embodiment, treatment of juvenile rheumatoid arthritis in a subject comprises inducing and maintaining remission of juvenile rheumatoid arthritis in a subject. In another embodiment, treatment of juvenile rheumatoid arthritis in a subject comprises maintaining remission of juvenile rheumatoid arthritis in a subject.

Those "in need of treatment" include mammals, such as humans, already having juvenile rheumatoid arthritis, including those in which the disease or disorder is to be prevented.

Various aspects of the invention are described in further detail herein.

The invention provides improved uses and compositions for treating juvenile rheumatoid arthritis with a TNFα inhibitor, *e*.*g*., a human TNFα antibody, or an antigen-binding portion thereof. Compositions and articles of manufacture, including kits, relating to the methods and uses for treating juvenile rheumatoid arthritis are also contemplated as part of the invention.

### 11. TNF Inhibitors

A TNFα inhibitor which is used in the methods and compositions of the invention includes any agent which interferes with TNFα activity. In a preferred embodiment, the TNFα inhibitor can neutralize TNFα activity, particularly detrimental TNFα activity which is associated with juvenile rheumatoid arthritis, and related complications and symptoms.

In one embodiment, the TNFα inhibitor used in the invention is an TNFα antibody (also referred to herein as a TNFα antibody), or an antigen-binding fragment thereof, including chimeric, humanized, and human antibodies. Examples of TNFα antibodies which may be used in the invention include, but not limited to, infliximab (Remlcade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (HUMIRA^{®} Abbott Laboratories, a human anti-TNF mAb, described in US 6,090,382 as D2E7). Additional TNF antibodies which may be used in the invention are described in U.S. Patent Nos. 6,593,458; 6,498,23.7; 6,451,983; and 6,448,380, each of which is incorporated by reference herein.

Other examples of TNFα inhibitors which may be used in the methods and compositions of the invention include etanercept (Enbrel, described in WO 91/03553 and WO 09/406476), soluble TNF receptor Type I, a pegylated soluble TNF receptor Type I (PEGs TNF-R1), p55TNPR1gG (Lenercept), and recombinant TNF binding protein (r-TBP-I) (Serono).

In one embodiment, the term "TNFα inhibitor" excludes infliximab. In one embodiment, the term "TNFα inhibitor" excludes adalimumab. In another embodiment, the term "TNFα inhibitor" excludes adalimumab and infliximab.

In one embodiment, the term "TNFα inhibitor" excludes etanercept, and, optionally, adalimumab, infliximab, and adalimumab and infliximab.

In one embodiment, the term "TNFα antibody" excludes infliximab. In one embodiment, the term "TNFα antibody" excludes adalimumab. In another embodiment, the term "TNFα antibody" excludes adalimumab and infliximab.

In one embodiment, the invention features uses and composition for treating or determining the efficacy of a TNFα inhibitor for the treatment of juvenile rheumatoid arthritis, wherein the TNFα antibody is an isolated human antibody, or antigen-binding portion thereof, that binds to human TNF_{α} with high affinity and a low off rate, and also has a high neutralizing capacity. Preferably, the human antibodies used in the invention are recombinant, neutralizing human anti-hTNFα antibodies, The most preferred recombinant, neutralizing antibody of the invention is referred to herein as D2E7, also referred to as HUMIRA^{®} or adalimumab (the amino acid sequence of the D2E7 VL region is shown in SEQ ID NO: 1; the amino acid sequence of the D2E7 VH region is shown in SEQ ID NO: 2). The properties of D2E7 (adalimumab / HUMIRA^{®}) have been described in Salfeld et al., U.S. Patent Nos. 6,090,382, 6,258,562, and 6,509,015, which are each incorporated by reference herein. The methods of the invention may also be performed using chimeric and humanized murine anti-hTNFα antibodies which have undergone clinical testing for treatment of rheumatoid arthritis (see *e.g*., Elliott, M.J., et al.(1994) Lancet 344:1125-1127; Elliot, M.J., et al. (1994) Lancet 344:1105-1110; Rankin, E.C., et al. (1995) Br. J. Rheumatol. 34:334-342).

In one embodiment, the method of the invention includes determining the efficacy of D2E7 antibodies and antibody portions, D2E7-related antibodies and antibody portions, or other human antibodies and antibody portions with equivalent properties to D2E7, such as high affinity binding to hTNFα with low dissociation kinetics and high neutralizing capacity, for the treatment of juvenile rheumatoid arthritis. In one embodiment, the invention provides treatment with an isolated human antibody, or an antigen-binding portion thereof, that dissociates from human TNFα with a K_{d} of 1 x 10⁻⁸ M or less and a K_{off} rate constant of 1 x 10⁻³ s⁻¹ or less, both determined by *surface plasmon resonance, and neutralizes human TNFα cytotoxicity in a standard in vitro* L929 assay with an IC₅₀ of 1 x 10⁻⁷ M or less. More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ of 1 x 10⁻⁹ M or less and still more preferably with an IC₅₀ of 1 x 10⁻¹⁰ M or less. In a preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to treating juvenile rheumatoid arthritis by administering human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the K_{off}. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the K_{off}. Accordingly, a consensus motif for the D2E7 VH CDR3 comprises the amino acid sequence: V-S-Y-L-S-T-A-S-S-L-D-(Y/N) (SEQ ID NO: 4), Moreover, as demonstrated in Example 2 of U.S. Patent No. 6,090,382, the CDR3 domain of the D2E7 heavy and light chains is amenable to substitution with a single alanine residue (at position 1, 4, 5, 7 or 8 within the VL CDR3 or at position 2, 3, 4, 5, 6, 8, 9, 10 or 11 within the VH CDR3) without substantially affecting the K_{off}. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) *(see* U.S. Patent No. 6,090,382).

Accordingly, in another embodiment, the antibody or antigen-binding portion thereof preferably contains the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ *s⁻¹* or less. Even more preferably, the antibody, or antigen-binding portion thereof, dissociates from human TNFα with a K_{off} of 1 x 10⁻⁴ s*⁻¹* or less.

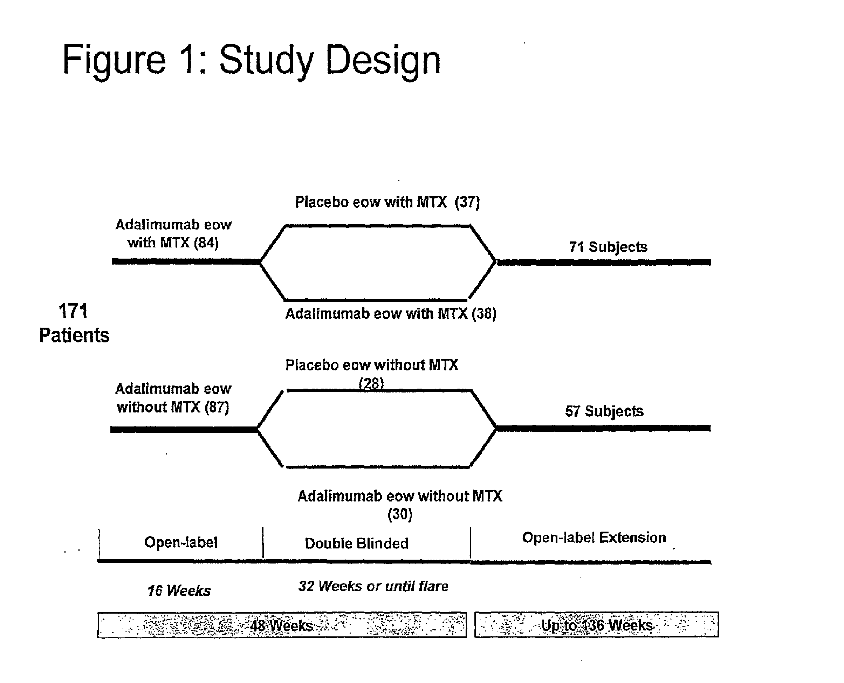

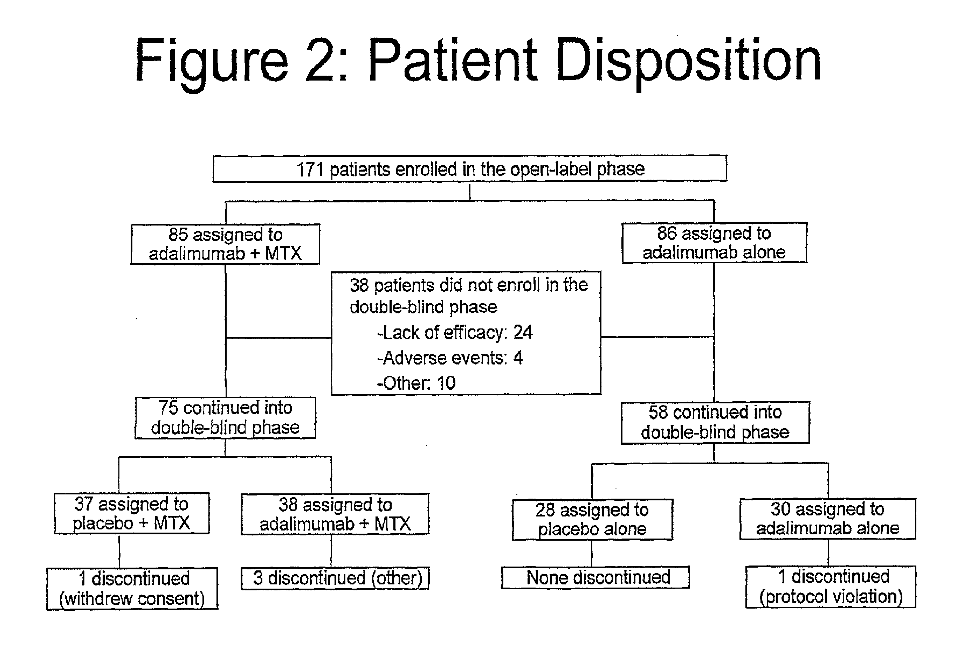

In yet another embodiment, the antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 (i.e., the D2E7 VL CDR2) and the HCVR further has a CDR2 domain comprising the amino acid sequence of SEQ ID NO: 6 (i.e., the D2E7 VH CDR2). Even more preferably, the LCVR further has CDR1 domain comprising the amino acid sequence of SEQ ID NO: 7 *(i.e.,* the D2E7 VL CDR1) and the HCVR has a CDR1 domain comprising the amino acid sequence of SEQ ID NO: 8 *(i.e.,* the D2E7 VH CDR1). The framework regions for VL preferably are from the VκI human germline family, more preferably from the A20 human germline Vk gene and most preferably from the D2E7 VL framework sequences shown in Figures 1A and 1B of U.S. Patent No. 6,090,382. The framework regions for VH preferably are from the V_{H}3 human germline family, more preferably from the DP-31 human germline VH gene and most preferably from the D2E7 VH framework sequences shown in Figures 2A and 2B of U.S. Patent No. 6,090,382.

Accordingly, in another embodiment, the antibody or antigen-binding portion thereof preferably contains a light chain variable region (LCVR) comprising the amino acid sequence of SEQ ID NO: 1 (*i.e.*, the D2E7 VL) and a *heavy* chain variable region (HCVR) comprising the amino acid sequence of SEQ ID NO: 2 (*i.e.,* the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the invention includes uses of an isolated human antibody, or an antigen-binding portions thereof, containing D2E7-related VL and VH CDR3 domains, For example, antibodies, or antigen-binding portions thereof, with a light chain variable region (LCVR) having a CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

The TNFα antibody used in the methods and compositions of the invention may be modified for improved treatment of juvenile rheumatoid arthritis. In some embodiments, the TNFα antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factor 3:4-10 (1992); EP 0 154 316; and EP 0 401 384 (each of which is incorporated by reference herein in its entirety). Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (CI-CIO) alkoxy- or aryloxy-polyethylene glycol.

Methods for preparing pegylated antibodies and antibody fragments of the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

Pegylated antibodies and antibody fragments may generally be used to treat juvenile rheumatoid arthritis by administration of the TNFα antibodies and antibody fragments described herein. Generally the pegylated antibodies and antibody fragments have increased half-life, as compared to the nonpegylated antibodies and antibody fragments. The pegylated antibodies and antibody fragments may be employed alone, together, or in combination with other pharmaceutical compositions.

In yet another embodiment of the invention, TNFα antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see *e.g.*, Canfield, S.M. and S.L. Morrison (1991) J. Exp. Med. 173:1483-1491; and Lund, J. et al. (1991) J. of Immunol. 147:2657-2662). Reduction in FcR binding ability of the antibody may also reduce other effector functions which rely on FcR interactions, such as opsonization and phagocytosis and antigen-dependent cellular cytotoxicity.

An antibody or antibody portion used in the methods of the invention can be derivatized or linked to another functional molecule (*e.g*., another peptide or protein). Accordingly, the antibodies and antibody portions of the invention are intended to include derivatized and otherwise modified forms of the human anti-hTNFα antibodies described herein, including immunoadhesion molecules. For example, an antibody or antibody portion of the invention can be functionally linked (by chemical coupling, genetic fusion, noncovalent association or otherwise) to one or more other molecular entities, such as another antibody (*e.g.*, a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more' antibodies (of the same type or of different types, *e.g.*, to create bispecific antibodies). Suitable crosslinkers include those that are heterobifunctional, having two distinctly reactive groups separated by an appropriate spacer (*e.g.*, m-maleimidobenzoyl-N-hydroxysuccinimide ester) or homobifunctional (*e.g.*, disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, used in the methods and compositions of the invention, can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express adalimumab (D2E7) or an adalimumab (D2E7)-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see *e.g.,* the "Vbase" human germline sequence database; see also Kabat, E.A,, et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of VH Segments with Different Hypervariable Loops" J. Mol. Biol. 227:776-798; and Cox, J.P.L. et al. (1994) "A Directory of Human Germ-line V78 Segments Reveals a Strong Bias in their Usage" Eur. J. Immunol. 24:827-836; the contents of each of which are expressly incorporated herein by reference). To obtain a DNA fragment encoding the heavy chain variable region of D2E7, or a D2E7-related antibody, a member of the V_{H}3 family of human germline VH genes is amplified by standard PCR. Most preferably, the DP-31 VH germline sequence is amplified. To obtain a DNA fragment encoding the light chain variable region of D2E7, or a D2E7-related antibody, a member of the V_{κ}I family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra,* using standard methods.

Once the germline VH and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Moreover, it should be noted that if the "germline" sequences obtained by PCR amplification encode amino acid differences in the framework regions from the true germline configuration (*i.e*., differences in the amplified sequence as compared to the true germline sequence, for example as a result of somatic mutation), it may be desireable to change these amino acid differences back to the true germline sequences (*i.e.*, "backmutation" of framework residues to the germline configuration).

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e.g.*, Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.,* Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e.g*., encoding the amino acid sequence (Gly₄-Ser)₃, such that the VH and VL sequences can be expressed as a contiguous single-chain protein, with the VL and VH regions joined by the flexible linker (see *e.g*., Bird et al. (1988) Science 242:423-426; Huston et al. (1988) Proc. Natl. Acad, Sci. USA 85:5879-5883; McCafferty et al., Nature (1990) 348:552-554).

To express the antibodies, or antibody portions used in the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e.g.*, ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2E7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e*., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (*e.g.* polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.*, the adenovirus major late promoter (AdMLP)) and polyoma. For further description of viral regulatory elements, and sequences thereof, see *e.g.*, U.S. Patent No. 5,168,062 by Stinski, U.S. Patent No. 4,510,245 by Bell et al. and U.S. Patent No. 4,968,615 by Schaffner et al.

In addition to the antibody chain genes and regulatory sequences, the recombinant expression vectors used in the invention may carry additional sequences, such as sequences that regulate replication of the vector in host cells (*e.g.*, origins of replication) and selectable marker genes. The selectable marker gene facilitates selection of host cells into which the vector has been introduced (see *e.g.*, U.S. Patents Nos. 4,399,216, 4,634,665 and 5,179,017, all by Axel *et al.*). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the *neo* gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g*., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g.*, as described in R.J. Kaufman and P.A. Sharp (1982) Mol. Biol. 159:601-621), NSO myeloma cells, COS cells and SP2 cells, When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecule. It is understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

In view of the foregoing, nucleic acid, vector and host cell compositions that can be used for recombinant expression of the antibodies and antibody portions used in the invention include nucleic acids, and vectors comprising said nucleic acids, comprising the human TNFα antibody adalimumab (D2E7). The nucleotide sequence encoding the D2E7 light chain variable region is shown in SEQ ID NO: 36. The CDR1 domain of the LCVR encompasses nucleotides 70-102, the CDR2 domain encompasses nucleotides 148-168 and the CDR3 domain encompasses nucleotides 265-291. The nucleotide sequence encoding the D2E7 heavy chain variable region is shown in SEQ ID NO: 37. The CDR1 domain of the HCVR encompasses nucleotides 91-105, the CDR2 domain encompasses nucleotides 148-198 and the CDR3 domain encompasses nucleotides 295-330. It will be appreciated by the skilled artisan that nucleotide sequences encoding D2E7-related antibodies, or portions thereof (*e.g.*, a CDR domain, such as a CDR3 domain), can be derived from the nucleotide sequences encoding the D2E7 LCVR and HCVR using the genetic code and standard molecular biology techniques.

Recombinant human antibodies of the invention in addition to D2E7 or an antigen binding portion thereof, or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (*e.g.*, the Pharmacia *Recombinant Phage Antibody System,* catalog no. 27-9400-01; and the Stratagene *SurfZAP*^{™} phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) BiolTechnology 9:1370-1372; Hay et al. (1992) Hum Antibod Hybridomas 3:81-65; Huse et al. (1989) Science 246:1275-1281; McCafferty et al., Nature (1990) 348:552-554; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982.

In a preferred embodiment, to isolate human antibodies with high affinity and a low off rate constant for hTNFα, a murine anti-hTNFα antibody having high affinity and a low off rate constant for hTNFα (*e.g.*, MAK 195, the hybridoma for which has deposit number ECACC 87 050801) is first used to select human heavy and light chain sequences having similar binding activity toward hTNFα, using the epitope imprinting methods described in Hoogenboom et al., PCT Publication No. WO 93/06213. The antibody libraries used in this method are preferably scFv libraries prepared and screened as described in McCafferty et al., PCT Publication No. WO 92/01047, McCafferty et al., Nature (1990) 348:552-554; and Griffiths et al., (1993) EMBO J 12:725-734. The scFv antibody libraries preferably are screened using recombinant human TNFα as the antigen.

Once initial human VL and VH segments are selected, "mix and match" experiments, in which different pairs of the initially selected VL and VH segments are screened for hTNFα binding, are performed to select preferred VL/VH pair combinations. Additionally, to further improve the affinity and/or lower the off rate constant for hTNFα binding, the VL and VH segments of the preferred VL/VH pair(s) can be randomly mutated, preferably within the CDR3 region of VH and/or VL, in a process analogous to the *in vivo* somatic mutation process responsible for affinity maturation of antibodies during a natural immune response. This *in vitro* affinity maturation can be accomplished by amplifying VH and VL regions using PCR primers complimentary to the VH CDR3 or VL CDR3, respectively, which primers have been "spiked" with a random mixture of the four nucleotide bases at certain positions such that the resultant PCR products encode VH and VL segments into which random mutations have been introduced into the VH and/or VL CDR3 regions. These randomly mutated VH and VL segments can be rescreened for binding to hTNFα and sequences that exhibit high affinity and a low off rate for hTNFα binding can be selected.

Following screening and isolation of an anti-hTNFα antibody of the invention from a recombinant immunoglobulin display library, nucleic acid encoding the selected antibody can be recovered from the display package (*e.g*., from the phage genome) and subcloned into other expression vectors by standard recombinant DNA techniques. If desired, the nucleic acid can be further manipulated to create other antibody forms of the invention (*e.g.*, linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in above.

Methods of isolating human neutralizing antibodies with high affinity and a low off rate constant for hTNFα are described in U.S. Patent Nos. 6,090,382, 6,258,562, and 6,509,015, each of which is incorporated by reference herein.

Antibodies, antibody-portions, and other TNFα inhibitors for use in the methods of the invention, can be incorporated into pharmaceutical compositions suitable for administration to a subject. Typically, the pharmaceutical composition comprises an antibody, antibody portion, or other TNFα inhibitor, and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody, antibody portion, or other TNFα inhibitor.

The compositions for use in the methods and compositions of the invention may be in a variety of forms. These include, for example, liquid, semi-solid and solid dosage forms, such as liquid solutions (*e.g.*, injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies or other TNFα inhibitors. The preferred mode of administration is parenteral (*e.g.*, intravenous, subcutaneous, intraperitoneal, intramuscular). In a preferred embodiment, the antibody or other TNFα inhibitor is administered by intravenous infusion or injection. In another preferred embodiment, the antibody or other TNFα inhibitor is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i.e.*, antibody, antibody portion, or other TNFα inhibitor) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In one embodiment, the invention includes pharmaceutical compositions comprising an effective TNFα inhibitor and a pharmaceutically acceptable carrier, wherein the effective TNFα inhibitor may be used to treat juvenile rheumatoid arthritis.

In one embodiment, the antibody or antibody portion for use in the methods of the invention is incorporated into a pharmaceutical formulation as described in PCT/IB03/04502 and U.S. Appln. No. 20040033228, incorporated by reference herein. This formulation includes a concentration 50 mg/ml of the antibody D2E7 (adalimumab.), wherein one pre-filled syringe contains 40 mg of antibody for subcutaneous injection.

The antibodies; antibody-portions, and other TNFα inhibitors of the present invention can be administered by a variety of methods known in the art, although for many therapeutic applications, the preferred route/mode of administration is parenteral, *e.g*., subcutaneous injection. In another embodiment, administration is via intravenous injection or infusion.

As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g.*, Sustained and Controlled Release Drug Delivery Systems, Robinson, ed., Dekker, Inc., New York, 1978.

In one embodiment, the TNFα antibodies and inhibitors used in the invention are delivered to a subject subcutaneously. In one embodiment, the subject administers the TNFα inhibitor, including, but not limited to, TNFα antibody, or antigen-binding portion thereof, to himself/herself.

The TNFα antibodies and inhibitors used in the invention may also be administered in the form of protein crystal formulations which include a combination of protein crystals encapsulated within a polymeric carrier to form coated particles. The coated particles of the protein crystal formulation may have a spherical morphology and be microspheres of up to 500 micro meters in diameter or they may have some other morphology and be microparticulates. The enhanced concentration of protein crystals allows the antibody of the invention to be delivered subcutaneously. In one embodiment, the TNFα antibodies of the invention are delivered via a protein delivery system, wherein one or more of a protein crystal formulation or composition, is administered to a subject with a TNFα-related disorder. Compositions and methods of preparing stabilized formulations of whole antibody crystals or antibody fragment crystals are also described in WO 02/072636, which is incorporated by reference herein. In one embodiment, a formulation comprising the crystallized antibody fragments described in PCT/IB03/04502 and U.S. Appln. No. 20040033228, incorporated by reference herein, are used to treat rheumatoid arthritis using the treatment methods of the invention.

In certain embodiments, an antibody, antibody portion, or other TNFα inhibitor of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions. In certain embodiments, an antibody or antibody portion for use in the methods of the invention is coformulated with and/or coadministered with one or more additional therapeutic agents, including an juvenile rheumatoid arthritis inhibitor or antagonist. For example, an anti-hTNFα antibody or antibody portion of the invention may be coformulated and/or coadministered with one or more additional antibodies that bind other targets associated with TNFα related disorders (*e.g.*, antibodies that bind other cytokines or that bind cell surface molecules), one or more cytokines, soluble TNFα receptor (see *e.g.*, PCT Publication No. WO 94/06476) and/or one or more chemical agents that inhibit hTNFα production or activity (such as cyclohexane-ylidene derivatives as described in PCT Publication No. WO 93/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

The pharmaceutical compositions of the invention may include a "therapeutically effective amount" or a "prophylactically effective amount" of an antibody or antibody portion of the invention. A "therapeutically effective amount" refers to an amount effective, at dosages and for periods of time necessary, to achieve the desired therapeutic result. A therapeutically effective amount of the antibody, antibody portion, or other TNFα inhibitor may vary according to factors such as the disease state, age, sex, and weight of the individual, and the ability of the antibody, antibody portion, other TNFα inhibitor to elicit a desired response in the individual. A therapeutically effective amount is also one in which any toxic or detrimental effects of the antibody, antibody portion, or other TNFα inhibitor are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Additional description regarding methods and uses of the invention comprising administration of a TNFα inhibitor are described in Part III of this specification.

The invention also pertains to packaged pharmaceutical compositions or kits for administering the anti-TNF antibodies of the invention for the treatment of juvenile rheumatoid arthritis. In one embodiment of the invention, the kit comprises a TNFα inhibitor, such as an antibody and instructions for administration of the TNFα inhibitor for treatment of juvenile rheumatoid arthritis. The instructions may describe how, *e.g.*, subcutaneously, and when, *e.g.*, at week 0, week 2, week 4, etc., the different doses of TNFα inhibitor shall be administered to a subject for treatment.

Another aspect of the invention pertains to kits containing a pharmaceutical composition comprising a TNFα inhibitor, such as an antibody, and a pharmaceutically acceptable carrier and one or more pharmaceutical compositions each comprising an additional therapeutic agent useful for treating juvenile rheumatoid arthritis, and a pharmaceutically acceptable carrier. Alternatively, the kit comprises a single pharmaceutical composition comprising an anti-TNFα antibody, one or more drugs useful for treating juvenile rheumatoid arthritis, and a pharmaceutically acceptable carrier. The instructions may describe how, *e.g.*, subcutaneously, and when, *e.g.*, at week 0, week 2, week 4, etc., the different doses of TNFα inhibitor and/or the additional therapeutic agent shall be administered to a subject for treatment.

The kit may contain instructions for dosing of the pharmaceutical compositions for the treatment of juvenile rheumatoid arthritis. Additional description regarding articles ofmanufacture of the invention are described in subsection III.

The package or kit alternatively can contain the TNFα inhibitor and it can be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### III. Uses and Compositions for Treating JRA

Tumor necrosis factor has been implicated in the pathophysiology of juvenile arthritis, including juvenile rheumatoid arthritis (Grom et al. (1996) Arthritis Rheum. 39:1703; Mangge et al. (1995) Arthritis Rheum. 8:211). The invention provides methods for determining the efficacy of a TNFα inhibitor, *e.g.*, a TNFα antibody, or antigen-binding portion thereof, to treat juvenile rheumatoid arthritis.

The term "juvenile rheumatoid arthritis" or "JRA" as used herein refers to a chronic, inflammatory disease which occurs before age 16 that may cause joint or connective tissue damage. JRA is also referred to as juvenile chronic polyarthritis and Still's disease. JRA is also referred to as juvenile idiopathic arthritis (JIA).

JRA causes joint inflammation and stiffness for more than 6 weeks in a child of 16 years of age or less. Inflammation causes redness, swelling, warmth, and soreness in the joints. Any joint can be affected and inflammation may limit the mobility of affected joints. One type of JRA can also affect the internal organs.

JRA is often classified into three types by the number of joints involved, the symptoms, and the presence or absence of certain antibodies found by a blood test. These classifications help the physician determine how the disease will progress and whether the internal organs or skin is affected. The classifications of JRA include the following
a. Pauciarticular JRA, wherein the patient has four or fewer joints are affected. Pauciarticular is the most common form of JRA, and typically affects large joints, such as the knees.
b. Polyarticular JRA, wherein five or more joints are affected. The small joints, such as those in the hands and feet, are most commonly involved, but the disease may also affect large joints.
c. Systemic JRA is characterized by joint swelling, fever, a light skin rash, and may also affect internal organs such as the heart, liver, spleen, and lymph nodes. Systemic JRA is also referred to as it Still's disease. A small percentage of these children develop arthritis in many joints and can have severe arthritis that continues into adulthood.

In one embodiment, the invention provides a method for preventing a flare-up associated with juvenile rheumatoid arthritis (JRA) in a subject comprising administering a TNFα antibody, or antigen-binding portion thereof, to a subject, such that a flare up is prevented. The invention also provides a use of a TNFα antibody, or antigen-binding portion thereof, in the manufacture of a medicament for preventing a flare-up associated with juvenile rheumatoid arthritis (JRA). In one embodiment, the TNFα antibody, or antigen-binding portion thereof, is administered with methotrexate. In one embodiment, the TNFα antibody, or antigen-binding portion thereof, is administered without methotrexate. In one embodiment, the TNFα antibody, or antigen-binding portion thereof, is administered at a dose of 24 mg/M² BSA.

A flare-up or flare associated with JRA describes a worsening of a symptom(s) associated with JRA (as compared to a remission or disappearance of a symptom(s)). The most common symptom of JRA is persistent joint swelling, pain, and stiffness that are typically worse in the morning or after a nap. The pain may limit movement of the affected joint. JRA commonly affects the knees and joints in the hands and feet. One of the earliest signs of JRA may be limping in the morning because of an affected knee. Besides joint symptoms, children with systemic JRA may have a high fever and a light pink rash. The rash and fever may appear and disappear very quickly. Systemic JRA also may also cause the lymph nodes located in the neck and other parts of the body to swell. In some cases, internal organs including the heart and very rarely the lungs may be involved. In one embodiment, the disease flare criteria includes a worsening of >30% in 3 of the 6 core criteria (from baseline of double-blind period), no fewer than two active joints, and improvement of 30% in no more than one criteria.

In one embodiment, the invention provides a combination method of preventing flare-ups associated.with JRA comprising administering a TNFα antibody, or antigen-binding portion thereof, and methotrexate. In one embodiment, the flare-up is prevented for at least 20 weeks when the subject is administered the TNFα antibody, or antigen-binding portion thereof, and methotrexate. In another embodiment, the flare-up is prevented for 21 weeks, for 22 weeks, for 23 weeks, for 24 weeks, for 25 weeks, for 26 weeks, for 27 weeks, for 28 weeks, for 29 weeks, for 30 weeks, for 31 weeks, for 32 weeks, or at least for 32 weeks.

In one embodiment, the invention provides a monotherapy method of preventing flare-ups associated with JRA comprising administering a TNFα antibody, or antigen-binding portion thereof. In one embodiment, the flare-up is prevented for at least 14 weeks when the subject is administered the TNFα antibody, or antigen-binding portion thereof. In another embodiment, the flare-up is prevented for 15 weeks, for 16 weeks, for 17 weeks, for 18 weeks, for 19 weeks, for 20 weeks, for 21 weeks, for 22 weeks, for 23 weeks, for 24 weeks, for 25 weeks, for 26 weeks, for 27 weeks, for 28 weeks, for 29 weeks, for 30 weeks, for 31 weeks, for 32 weeks, or at least for 32 weeks.

In one embodiment, the TNFα antibody, or antigen-binding portion thereof, is administered subcutaneously to the subject to prevent flare-ups associated with JRA.

The TNFα antibody, or an antigen-binding portion thereof, may be administered to the subject on a biweekly dosing regimen. In one embodiment, biweekly dosing includes a dosing regimen wherein doses of a TNFα inhibitor are administered to a subject every other week beginning at week 0. In one embodiment, biweekly dosing includes a dosing regimen where doses of a TNFα inhibitor are administered to a subject every other week consecutively for a given time period, *e.g.*, 4 weeks, 8 weeks, 16, weeks, 24 weeks, 26 weeks, 32 weeks, 36 weeks, 42 weeks, 48 weeks, 52 weeks, 56 weeks, etc. In one embodiment, the methods of the invention comprising administering a TNFα antibody, or an antigen-binding portion thereof, on a biweekly dosing regimen subcutaneously. Biweekly dosing regimens can be used to treat disorders in which TNFα activity is detrimental, and are further described in US Appln. No. 10/163657 (US 20030235585), incorporated by reference herein. In one embodiment, biweekly dosing includes a dosing regimen where doses of a TNFα inhibitor are administered to a subject every other week consecutively for a given time period, *e.g.*, 4 weeks, 8 weeks, 16, weeks, 24 weeks, 26 weeks, 32 weeks, 36 weeks, 42 weeks, 48 weeks, 52 weeks, 56 weeks, etc.

In one embodiment, treatment of JRA is achieved by administering a human TNFα antibody, or an antigen-binding portion thereof, to a subject having JRA, wherein the human TNFα antibody, or an antigen-binding portion thereof, is administered on a biweekly dosing regimen. In one embodiment, the human TNFα antibody, or an antigen-binding portion thereof, is administered in a dose of about 24 mg Ada/M² BSA. In one embodiment, the human TNFα antibody, or an antigen-binding portion thereof, is adalimumab.

Methods of treatment described herein may include administration of a TNFα inhibitor to a subject to achieve a therapeutic goal, *e.g.*, improvement in PedACR response, swollen joint count (SJC), time until flare-up, and tender joint count (TJC). Also included in the scope of the invention are uses of a TNFα inhibitor in the manufacture of a medicament to achieve a therapeutic goal, *e.g*., improvement in PedACR response, swollen joint count (SJC), time until flare-up, and tender joint count (TJC). Thus, where methods are described herein, it is also intended to be part of this invention that the use of the TNFα inhibitor in the manufacture of a medicament for the purpose of the method is also considered within the scope of the invention. Likewise, where a use of a TNFα inhibitor in the manufacture of a medicament for the purpose of achieving a therapeutic goal is described, methods of treatment resulting in the therapeutic goal are also intended to be part of the invention.

In one embodiment, the invention provides a method of treating JRA in a subject comprising administering a human TNFα antibody, or antigen-binding portion thereof, *e.g.*, adalimumab, to the subject at week 0 on a biweekly dosing regimen. In one embodiment, the human TNFα antibody, or antigen-binding portion thereof, is administered subcutaneously. In one embodiment, JRA is treated by administering a human TNFα antibody, or antigen-binding portion thereof, on biweekly dosing regimen for at least about 12 weeks, 13 weeks, 14 weeks, 15 weeks, for 16 weeks, for 17 weeks, for 18 weeks, for 19 weeks, for 20 weeks, for 21 weeks, for 22 weeks, for 23 weeks, for 24 weeks, for 25 weeks, for 26 weeks, for 27 weeks, for 28 weeks, for 29 weeks, for 30 weeks, for 31 weeks, for 32 weeks, 33 weeks, 34 weeks, 35 weeks, 36 weeks, 37 weeksn, 38 weeks, 39 weeks, 40 weeks, 41 weeks, 42 weeks, 43 weeks, 44 weeks, 45 weeks, 46 weeks, 47 weeks, 48 weeks, or at least about 48 weeks.

Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Dosage regimens described herein may be adjusted to provide the optimum desired response, *e.g.*, maintaining remission of juvenile rheumatoid arthritis, in consideration of the teachings herein. It is to be noted that dosage values may vary with the type and severity of juvenile rheumatoid arthritis. It is to be further understood that for any particular subject, specific dosage regimens may be adjusted over time according to the teachings of the specification and the individual need and the professional judgment of the person administering or supervising the administration of the compositions, and that dosage amounts and ranges set forth herein are exemplary only and are not intended to limit the scope or practice of the claimed invention.

Examples of other methods and uses of TNFα inhibitors for the treatment of JRA are also described in 60/793737, ffiled April 19 2006, and 60/798149, filed May 4, 2006, incorporated in their entirely herein.

### Articles of Manufacture

The invention also provides a packaged pharmaceutical composition wherein the TNFα inhibitor, *e.g.*, TNFα antibody, is packaged within a kit or an article of manufacture. The kit or article of manufacture of the invention contains materials useful for the treatment, including induction and/or remission, prevention and/or diagnosis of juvenile rheumatoid arthritis. The kit or article of manufacture comprises a container and a label or package insert or printed material on or associated with the container which provides information regarding use of the TNFα inhibitor, *e.g.*, a TNFα antibody, for the treatment of juvenile rheumatoid arthritis.

A kit or an article of manufacture refers to a packaged product comprising components with which to administer a TNFα inhibitor for treatment of a juvenile rheumatoid arthritis. The kit preferably comprises a box or container that holds the components of the kit. The box or container is affixed with a label or a Food and Drug Administration approved label, including a protocol for administering the TNFα inhibitor. The box or container holds components of the invention which are preferably contained within plastic, polyethylene, polypropylene, ethylene, or propylene vessels. The vessels can be capped-tubes or bottles. The kit can also include instructions for administering the TNFα antibody of the invention. In one embodiment the kit of the invention includes the formulation comprising the human antibody adalimumab (or D2E7), as described in PCT/IB03/04502 and U.S. Appln. No. 10/222140, incorporated by reference herein.

The term "package insert" is used to refer to instructions customarily included in commercial packages of therapeutic products, that contain information about the indications, usage, dosage, administration, contraindications and/or warnings concerning the use of such therapeutic products.

In one embodiment, the article of manufacture of the invention comprises (a) a first container with a composition contained therein, wherein the composition comprises a TNFα antibody; and (b) a package insert indicating that the TNFα antibody may be used for reducing signs and symptoms and inducing and maintaining remission of juvenile rheumatoid arthritis. In a preferred embodiment, the label or package insert indicates that the TNFα inhibitor, *e.g.*, a TNFα antibody, is used for inducing and maintaining remission juvenile rheumatoid arthritis.

Suitable containers for the TNFα inhibitor, *e.g.*, a TNFα antibody, include, for example, bottles, vials, syringes, pens, etc. The containers may be formed from a variety of materials such as glass or plastic, The container holds a composition which is by itself or when combined with another composition effective for treating, preventing and/or diagnosing the condition and may have a sterile access port.

In one embodiment, the article of manufacture comprises a TNFα inhibitor, *e.g.*, a TNFα antibody, and a label which indicates to a subject who will be administering the TNFα inhibitor about using the TNFα inhibitor for the treatment of juvenile rheumatoid arthritis, including polyarticular JRA. The label may be anywhere within or on the article of manufacture. In one embodiment, the article of manufacture comprises a container, such as a box, which comprises the TNFα inhibitor and a package insert or label providing information pertaining to use of the TNFα inhibitor for the treatment of juvenile rheumatoid arthritis. In another embodiment, the information is printed on a label which is on the outside of the article of manufacture, in a position which is visible to prospective purchasers.

In one embodiment, the package insert of the invention informs a reader, including a subject, *e.g.*, a purchaser, who will be administering the TNFα inhibitor for treatment, that the TNFα inhibitor, *e.g*., a TNFα antibody such as adalimumab, is an indicated treatment of juvenile rheumatoid arthritis, including polyarticular JRA.

In one embodiment, the package insert of the invention describes certain therapeutic benefits of the TNFα antibody, *e.g*., adalimumab, including specific symptoms of juvenile rheumatoid arthritis which may be reduced by using the TNFα antibody, *e.g*., adalimumab. It should be noted that the package insert may also contain information pertaining to other disorders which are treatable using the TNFα antibody, *e.g*., adalimumab. Information described herein which is provided in a package insert and pertains to other disorders, *i.e*., diseases other than juvenile rheumatoid arthritis, is also included within the scope of the invention.

In another embodiment, the package insert of the invention describes the dose and administration of adalimumab, for the treatment of juvenile rheumatoid arthritis. The label may indicate that therapy includes a 24 mg Ada/M² BSA dose at week 0, which is administered every other week subcutaneously. The label may also indicate that the maintenance dosing for the treatment of juvenile rheumatoid arthritis with adalimumab is 24 mg Ada/M² BSA every other week. The label may also indicate that some patients with juvenile rheumatoid arthritis may derive additional benefit by increasing frequency to 24 mgAda/M² BSA every week. In another embodiment, the package insert of the invention indicates that adalimumab is administered by subcutaneous injection.

The package insert of the invention may also provide information to subjects who will be receiving adalimumab regarding combination uses for both safety and efficacy purposes. The package insert of the invention may contain warnings and precautions regarding the use of the TNFα inhibitor, *e.g*., a TNFα antibody such as adalimumab. In one embodiment, the information provided in the label describes certain adverse events identified during studies of the efficacy and safety of the TNFα inhibitor.

The label of the invention may contain information regarding the use of the TNFα inhibitor, *e.g*., a TNFα antibody such as adalimumab, in clinical studies for juvenile rheumatoid arthritis. In one embodiment, the label of the invention describes the studies described herein as the Examples, either as a whole or in portion.

In one embodiment of the invention, the kit comprises a TNFα inhibitor, such as an antibody, an second pharmaceutical composition comprising an additional therapeutic agent, and instructions for administration of both agents for the treatment of juvenile rheumatoid arthritis. The instructions may describe how, *e.g*., subcutaneously, and when, *e.g*., at week 0, week 2, and biweekly thereafter, doses of TNFα antibody and/or the additional therapeutic agent shall be administered to a subject for treatment.

Another aspect of the invention pertains to kits containing a pharmaceutical composition comprising an anti-TNFα antibody and a pharmaceutically acceptable carrier and one or more additional pharmaceutical compositions each comprising a drug useful for treating a TNFα related disorder and a pharmaceutically acceptable carrier. Alternatively, the kit comprises a single pharmaceutical composition comprising an anti-TNFα antibody, one or more drugs useful for treating a TNFα related disorder and a pharmaceutically acceptable carrier. The kits further contain instructions for dosing of the pharmaceutical compositions for the treatment of a TNFα related disorder.

The package or kit alternatively may contain the TNFα inhibitor and it may be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### Additional therapeutic agents

TNFα inhibitors, including antibodies, used in the methods and compositions of the invention, or antigen binding portions thereof can be used alone or in combination to treat such diseases. It should be understood that the antibodies or antigen binding portion thereof can be used alone or in combination with an additional agent, e.g., a therapeutic agent, said additional agent being selected by the skilled artisan for its intended purpose. For example, the additional agent can be a therapeutic agent art-recognized as being useful to treat the disease or condition being treated by the antibody of the present invention. The additional agent also can be an agent that imparts a beneficial attribute to the therapeutic composition e.g., an agent which effects the viscosity of the composition.

It should further be understood that the combinations which are to be included within this invention are those combinations useful for their intended purpose. The agents set forth below are illustrative for purposes and not intended to be limited. The combinations, which are part of this invention, can be the antibodies of the present invention and at least one additional agent selected from the lists below. The combination can also include more than one additional agent, e.g., two or three additional agents if the combination is such that the formed composition can perform its intended function.

Binding proteins described herein may be used in combination with additional therapeutic agents such as a Disease Modifying Anti-Rheumatic Drug (DMARD) or a Nonsteroidal Antiinflammatory Drug (NSAID) or a steroid or any combination thereof. Preferred examples of a DMARD are hydroxychloroquine, leflunomide, methotrexate, parenteral gold, oral gold and sulfasalazine. Preferred examples of non-steroidal anti-inflammatory drug(s) also referred to as NSAIDS include drugs like ibuprofen. Other preferred combinations are corticosteroids including prednisolone; the well known side effects of steroid use can be reduced or even eliminated by tapering the steroid dose required when treating patients in combination with the anti-**XXX** antibodies of this invention. Non-limiting examples of therapeutic agents for rheumatoid arthritis with which an antibody, or antibody portion, of the invention can be combined include the following: cytokine suppressive anti-inflammatory drug(s) (CSAIDs); antibodies to or antagonists of other human cytokines or growth factors, for example, TNF, LT, IL-1, IL-2, IL-3, IL-4, IL-5, IL-6, IL-7, IL-8, IL-15, IL-16, IL-18, IL-21, IL-23, interferons, EMAP-II, GM-CSF, FGF, and PDGF. Antibodies of the invention, or antigen binding portions thereof, can be combined with antibodies to cell surface molecules such as CD2, CD3, CD4, CD8, CD25, CD28, CD30, CD40, CD45, CD69, CD80 (B7.1), CD86 (B7.2), CD90, CTLA or their ligands including CD154 (gp39 or CD40L).

Preferred combinations of therapeutic agents may interfere at different points in the autoimmune and subsequent inflammatory cascade; preferred examples include TNF antagonists such as soluble p55 or p75 TNF receptors, derivatives, thereof, (p75TNFR1gG (Enbrel^{™}) or p55TNFR1gG (Lenercept), chimeric, humanized or human TNF antibodies, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (Humira^{®} Abbott Laboratories, a human anti-TNF mAb, described in US 6,090,382 as D2E7), . Additional TNF antibodies which can be used in the invention are described in U.S. Patent Nos. 6,593,458; 6,498,237; 6,451,983; and 6,448,380, each of which is incorporated by reference herein. Other combinations including TNFα converting enzyme (TACE) inhibitors; IL-1 inhibitors (Interleukin-1-converting enzyme inhibitors, IL-1RA etc.) may be effective for the same reason. Other preferred combinations include Interleukin 11. Yet another preferred combination are other key players of the autoimmune response which may act parallel to, dependent on or in concert with TNF function; especially preferred are IL-18 antagonists including IL-18 antibodies or soluble IL-18 receptors, or IL-18 binding proteins. It has been shown that TNF and IL-18 have overlapping but distinct functions and a combination of antagonists to both may be most effective. Yet another preferred combination are non-depleting anti-CD4 inhibitors. Yet other preferred combinations include antagonists of the co-stimulatory pathway CD80 (B7.1) or CD86 (B7.2) including antibodies, soluble receptors or antagonistic ligands.

The antibodies of the invention, or antigen binding portions thereof, may also be combined with agents, such as methotrexate, 6-MP, azathioprine sulphasalazine, mesalazine, olsalazine chloroquinine/hydroxychicroquine, pencillamine, aurothiomalate (intramuscular and oral), azathioprine, cochicine, corticosteroids (oral, inhaled and local injection), beta-2 adrenoreceptor agonists (salbutamol, terbutaline, salmeteral), xanthines (theophylline, aminophylline), cromoglycate, nedocromil, ketotifen, ipratropium and oxitropium, cyclosporin, FK506, rapamycin, mycophenolate mofetil, leflunomide, NSAIDs, for example, ibuprofen, corticosteroids such as prednisolone, phosphodiesterase inhibitors, adensosine agonists, antithrombotic agents, complement inhibitors, adrenergic agents, agents which interfere with signalling by proinflammatory - cytokines such as TNFα or IL-1 (e.g. IRAK, NIK, IKK, p38 or MAP kinase inhibitors), IL-1β converting enzyme inhibitors, TNFα converting enzyme (TACE) inhibitors, T-cell signalling inhibitors such as kinase inhibitors, metalloproteinase inhibitors, sulfasalazine, azathioprine, 6-mercaptopurines, angiotensin converting enzyme inhibitors, soluble cytokine receptors and derivatives thereof (e.g. soluble p55 or p75 TNF receptors and the derivatives p75TNFRIgG (Enbrel^{™} and p55TNFRIgG (Lenercept)), sIL-1RI, sIL-1RII, sIL-6R), antiinflammatory cytokines (e.g. IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone hcl, hydrocodone bitartrate/apap, diclofenac sodium/misoprostol, fentanyl, anakinra, human recombinant, tramadol hcl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline hcl, sulfadiazine, oxycodone hcl/acetaminophen, olopatadine hcl, misoprostol, naproxen sodium, omeprazole, cyclophosphamide, rituximab, IL-1 TRAP, MRA, CTLA4-IG, IL-18 BP, anti-IL-18, Anti-IL15, BIRB-796, SCIO-469, VX-702, AMG-548, VX-740, Roflumilast, IC-485, CDC-801, and Mesopram. Preferred combinations include methotrexate or leflunomide and in moderate or severe rheumatoid arthritis cases, cyclosporine.

Nonlimiting additional agents which can also be used in combination with an TNF antibody, or antigen-binding portion thereof, to treat rheumatoid arthritis, including JRA, include, but are not limited to, the following: non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2/infliximab (chimeric anti-TNFα antibody; Centocor); 75 kdTNFR-IgG/etanercept (75 kD TNF receptor-IgG fusion protein; Immunex; see *e.g.,* Arthritis & Rheumatism (1994) Vol. 37, S295; J. Invest. Med. (1996) Vol. 44, 235A); 55 kdTNF-IgG (55 kD TNF receptor-IgG fusion protein; Hoffmann-LaRoche); IDEC-CE9.1/SB 210396 (non-depleting primatized anti-CD4 antibody; IDEC/SmithKline; see *e.g.,* Arthritis & Rheumatism (1995) Vol. 38, S185); DAB 486-IL-2 and/or DAB 389-IL-2 (IL-2 fusion proteins; Seragen; see e.g., Arthritis & Rheumatism (1993) Vol. 36, 1223); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (*e.g*., agonist antibodies); IL-1RA (IL-1 receptor antagonist; Synergen/Amgen); anakinra (Kineret^{®}/Amgen); TNF-bp/s-TNF (soluble TNF binding protein; see *e.g.,* Arthritis & Rheumatism (1996) Vol. 39, No. 9 (supplement), S284; Amer. J. Physiol. - Heart and Circulatory Physiology (1995) Vol. 268, pp. 37-42); R973401 (phosphodiesterase Type IV inhibitor; see *e.g.,* Arthritis & Rheumatism (1996) Vol. 39, No. 9 (supplement), S282); MK-966 (COX-2 Inhibitor; see *e.g.,* Arthritis & Rheumatism (1996) Vol. 39, No. 9 (supplement), S81); Iloprost (see *e.g*., Arthritis & Rheumatism (1996) Vol. 39, No. 9 (supplement), S82); methotrexate; thalidomide (see *e.g.,* Arthritis & Rheumatism (1996) Vol. 39, No. 9 (supplement), S282) and thalidomide-related drugs (*e.g*., Celgen); leflunomide (anti-inflammatory and cytokine inhibitor; see *e.g.,* Arthritis & Rheumatism (1996) Vol. 39, No. 9 (supplement), S131*;* Inflammation Research (1996) Vol. 45, pp. 103-107); tranexamic acid (inhibitor of plasminogen activation; see *e.g.,* Arthritis & Rheumatism (1996) Vol. 39, No. 9 (supplement), S284); T-614 (cytokine inhibitor; see *e.g.,* Arthritis & Rheumatism (1996) Vol. 39, No. 9 (supplement), S282); prostaglandin E1 (see *e.g.,* Arthritis & Rheumatism (1996) Vol. 39, No. 9 (supplement), S282); Tenidap (non-steroidal anti-inflammatory drug; see *e.g.,* Arthritis & Rheumatism (1996) Vol. 39, No. 9 (supplement), S280); Naproxen (non-steroidal anti-inflammatory drug; see *e.g*., Neuro Report (1996) Vol. 7, pp. 1209-1213); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine (see *e.g.,* Arthritis & Rheumatism (1996) Vol. 39, No. 9 (supplement), S281); Azathioprine (see *e.g.,* Arthritis & Rheumatism (1996) Vol. 39, No. 9 (supplement), S281); ICE inhibitor (inhibitor of the enzyme interleukin-1β converting enzyme); zap-70 and/or lck inhibitor (inhibitor of the tyrosine kinase zap-70 or lck); VEGF inhibitor and/or VEGF-R inhibitor (inhibitors of vascular endothelial cell growth factor or vascular endothelial cell growth factor receptor; inhibitors of angiogenesis); corticosteroid anti-inflammatory drugs (*e.g*., SB203580); TNF-convertase inhibitors; anti-IL-12 antibodies; anti-IL-18 antibodies; interleukin-11 (see *e.g.,* Arthritis & Rheumatism (1996) Vol. 39, No. 9 (supplement), S296); interleukin-13 (see *e.g.,* Arthritis & Rheumatism (1996) Vol. 39, No. 9 (supplement), S308); interleukin -17 inhibitors (see *e.g.,* Arthritis & Rheumatism (1996) Vol. 39, No. 9 (supplement), S120); gold; penicillamine; chloroquine; chlorambucil; hydroxychloroquine; cyclosporine; cyclophosphamide; total lymphoid irradiation; anti-thymocyte globulin; anti-CD4 antibodies; CD5-toxins; orally-administered peptides and collagen; lobenzarit disodium; Cytokine Regulating Agents (CRAs) HP228 and HP466 (Houghten Pharmaceuticals, Inc.); ICAM-1 antisense phosphorothioate oligo-deoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids; see e.g., DeLuca et al. (1995) Rheum. Dis. Clin. North Am. 21:759-777); auranofin; phenylbutazone; meclofenamic acid; flufenamic acid; intravenous immune globulin; zileuton; azaribine; mycophenolic acid (RS-61443); tacrolimus (FK-506); sirolimus (rapamycin); amiprilose (therafectin); cladribine (2-chlorodeoxyadenosine); methotrexate; antivirals; and immune modulating agents.

In one embodiment, the TNF antibody, or antigen-binding portion thereof, is administered in combination with one of the following agents for the treatment of rheumatoid arthritis: small molecule inhibitor of KDR (ABT-123), small molecule inhibitor of Tie-2; methotrexate; prednisone; celecoxib; folic acid; hydroxychloroquine sulfate; rofecoxib; etanercept; infliximab; leflunomide; naproxen; valdecoxib; sulfasalazine; methylpredhisolone; ibuprofen; meloxicam; methylprednisolone acetate; gold sodium thiomalate; aspirin; azathioprine; triamcinolone acetonide; propxyphene napsylate/apap; folate; nabumetone; diclofenac; piroxicam; etodolac; diclofenac sodium; oxaprozin; oxycodone hcl; hydrocodone bitartrate/apap; diclofenac sodium/misoprostol; fentanyl; anakinra, human recombinant; tramadol hcl; salsalate; sulindac; cyanocobalamin/fa/pyridoxine; acetaminophen; alendronate sodium; prednisolone; morphine sulfate; lidocaine hydrochloride; indomethacin; glucosamine sulfate/chondroitin; cyclosporine; arrtitriptyline hcl; sulfadiazine; oxycodone hcl/acetaminophen; olopatadine hcl; misoprostol; naproxen sodium; omeprazole; mycophenolate mofetil; cyclophosphamide; rituximab; IL-1 TRAP; MRA; CTLA4-IG; IL-18 BP; ABT-874; ABT-325 (anti-IL 18); anti-IL 15; BIRB-796; SCIO-469; VX-702; AMG-548; VX-740; Roflumilast; IC-485; CDC-801; and mesopram. In another embodiment, an TNF antibody, or antigen-binding portion thereof, is administered for the treatment of an TNF-related disorder in combination with one of the above mentioned agents for the treatment of rheumatoid arthritis.

The antibodies used in the methods and compositions of the invention, or antigen binding portions thereof, may also be combined with agents, such as alemtuzumab, dronabinol, Unimed, daclizumab, mitoxantrone, xaliproden hydrochloride, fampridine, glatiramer acetate, natalizumab, sinnabidol, a-immunokine NNSO3 ABR-215062, AnergiX.MS, chemokine receptor antagonists, BBR-2778, calagualine, CPI-1189, LEM (liposome encapsulated mitoxantrone), THC.CBD (cannabinoid agonist) MBP-8298, mesopram (PDE4 inhibitor), MNA-715, anti-IL-6 receptor antibody, neurovax, pirfenidone allotrap 1258 (RDP-1258), sTNF-R1, talampanel, teriflunomide,TGF-beta2, tiplimotide, VLA-4 antagonists (for example, TR-14035, VLA4 Ultrahaler, Antegran-ELAN/Biogen), interferon gamma antagonists, IL-4 agonists.

### IV. Efficacy of TNFα Inhibitor

The invention also provides methods for determining whether a TNFα inhibitor is effective at treating JRA in a subject. Such methods may be used to determine the efficacy of a TNFα inhibitor, including those which are unknown or unconfirmed to have such efficacy. Using the methods described herein, effective TNFα inhibitors may be determined or confirmed, and, subsequently, used in the method of treating JRA, as well as determining whether a TNFα inhibitor is effective for preventing flare-ups in a subject having RA.

In one embodiment, the invention provides a method for determining the efficacy of a TNFα antibody, or antigen-binding portion thereof, for the prevention of a flare-up associated with JRA in a subject, comprising determining a median time to flare of a patient population having JRA and who was administered the TNFα antibody, or antigen-binding portion thereof. In one embodiment, a median time to flare of at least about 14 weeks indicates that the TNFα antibody, or antigen-binding portion thereof, is an effective TNFα antibody, or antigen-binding portion thereof, for the prevention of flare associated with JRA. In one embodiment, the invention further comprises administering the effective TNFα antibody, or antigen-binding portion thereof, to a subject to prevent a flare-up associated with JRA.

In one embodiment, a flare-up occurrence rate of about 40% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 37% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 14% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 20% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 32% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 36% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 44% or less indicates that the TNFα antibody, or antigen-binding portion thereof, is an effective TNFα antibody, or antigen-binding portion thereof, for the prevention of the flare-up associated with JRA. Numbers intermediate to the above recited percentages, e.g., 15, 16, 17, 18,19, 21, 22, 23, 24, 25, 26, 27, 28,29, 30, 31, 33, 34, 35, 38, 39, 41, 42, 43%, as well as all other numbers recited herein, are also intended to be part of this invention. Ranges of values using a combination of any of the above recited values as upper and/or lower limits are intended to be included in the scope of the invention. For example, in one embodiment a CDAI score of less than 150 maintained in at least between 37 and 40% of the patient population indicates that the TNFα inhibitor is an effective TNFα inhibitor for the treatment of Crohn's disease in a subject.

The invention also provides a method of preventing a flare up in a subject having JRA comprising administering an effective TNFα antibody, or antigen-binding portion thereof, to a subject such that flare-up is prevented, wherein the effective TNFα antibody, or antigen-binding portion thereof, was previously identified as preventing a flare-up for at least about 14 weeks in a patient population having JRA.

The invention also provides a method for determining the efficacy of a TNFα antibody, or antigen-binding portion thereof, for the prevention of a flare-up associated with JRA in a subject, comprising determining a flare-up occurrence rate of a patient population having JRA and who was administered the TNFα antibody, or antigen-binding portion thereof, wherein a flare-up occurrence rate of about 43% or less indicates that the TNFα antibody, or antigen-binding portion thereof, is an effective TNFα antibody, or antigen-binding portion thereof, for the prevention of the flare-up associated with JRA. In one embodiment, the invention further comprises administering the effective TNFα antibody, or antigen-binding portion thereof, to a subject for the prevention of a flare-up associated with JRA.

The invention also includes a method of preventing a flare up in a subject having JRA comprising administering an effective TNFα antibody, or antigen-binding portion thereof, to a subject such that the flare-up is prevented, wherein the effective TNFα antibody, or antigen-binding portion thereof, was previously identified as preventing a flare-up in about 43% or less of a patient population having JRA. In one embodiment, a flare-up occurrence rate of about 40% or less indicates that the TNFα antibody, or antigen-binding portion thereof, is an effective TNFα antibody, or antigen-binding portion thereof, for the prevention of the flare-up associated with JRA. In another embodiment, a flare-up occurrence rate of about 37% or less indicates that the TNFα antibody, or antigen-binding portion thereof, is an effective TNFα antibody, or antigen-binding portion thereof, for the prevention of the flare-up associated with JRA.

The invention also includes a method for determining the efficacy of a TNFα inhibitor for the treatment of juvenile rheumatoid arthritis (JRA) in a subject comprising determining a Pediatric (Ped) ACR90 response of a patient population having JRA and who was administered the TNFα inhibitor, wherein a Ped ACR90 response achieved in at least about 15% of the patient population indicates that the TNFα inhibitor is an effective TNFα inhibitor for treating JRA. In one embodiment, the invention further comprises administering the effective TNFα inhibitor to a subject to treat JRA. The TNFα inhibitor may be administered either with or without methotrexate.

The Pediatric (Ped) ACR is a standardized set of definitions for remission and clinical improvement developed by the American College of Rheumatology (ACR) for measuring disease outcome and severity in patients with juvenile rheumatoid arthritis (see, for example, Giannini, et al. (1997) Arthritis Rheum. 40(7):1202-9). Improvement is denoted as either Ped ACR 20, ACR 50, ACR 70, or ACR 90 reflecting either an improvement from baseline in at least 3 of any 6 variables in the core set to the 20%, 50%, 70%, or 90% level, with no more than 1 of the remaining variables worsening by >30%. The variables in the core set consist of physician global assessment of disease activity, parent/patient assessment of overall well-being (each scored on a 10-cm Visual Analog Scale), functional ability, number of joints with active arthritis, number of joints with limited range of motion, and erythrocyte sedimentation rate. For example, an ACR20 response means clinical improvement indicated by a 20% improvement in the number of tender and swollen joints and a 20% improvement in at least three of five additional criteria.

In one embodiment, a Ped ACR90 response achieved in at least about 40% of the patient population indicates that the TNFα inhibitor is an effective TNFα inhibitor for treating JRA. In one embodiment, a Ped ACR90 response achieved in at least about 20% of the patient population indicates that the TNFα inhibitor is an effective TNFα inhibitor for treating JRA. In another embodiment, a Ped ACR90 response achieved in at least about 22% of the patient population indicates that the TNFα inhibitor is an effective TNFα inhibitor for treating JRA. In another embodiment, a Ped ACR90 response achieved in at least about 30% of the patient population indicates that the TNFα inhibitor is an effective TNFα inhibitor for treating JRA. In another embodiment, wherein a Ped ACR90 response achieved in at least about 39% of the patient population indicates that the TNFα inhibitor is an effective. In another embodiment, wherein a Ped ACR90 response achieved in at least about 40% of the patient population indicates that the TNFα inhibitor is an effective TNFα inhibitor for treating JRA. In another embodiment, a Ped ACR90 response achieved in at least about 42% of the patient population indicates that the TNFα inhibitor is an effective TNFα inhibitor for treating JRA. In another embodiment, wherein a Ped ACR90 response achieved in at least about 65% of the patient population indicates that the TNFα inhibitor is an effective. Numbers intermediate to the above recited percentages, e.g., 5,6,7,8,9, 10, 11, 12, 13, 14, 15, 16, 17,18,19,21,23,24,25,26,27,28,29,31, 32, 33, 34, 35, 36, 37, 38, 41, 43, 44, 45, 46, 47, 48, 49, 50, 51, 52, 53, 54, 55, 56, 57, 58, 59, 60, 61, 62, 63, 64%, as well as all other numbers recited herein, are also intended to be part of this invention. Ranges of values using a combination of any of the above recited values as upper and/or lower limits are intended to be included in the scope of the invention.

The invention also provides a method for treating JRA in a subject comprising administering an effective TNFα inhibitor to the subject such that JRA is treated, wherein the effective TNFα inhibitor was previously identified as achieving a Ped ACR90 response in at least about 15 % of a patient population having JRA.
In one embodiment, a Ped ACR90 response achieved in at least about 30% of the patient population indicates that the TNFα inhibitor is an effective TNFα inhibitor for treating JRA. the effective TNFα inhibitor was previously identified as achieving a Ped ACR90 response in at least about 20% of a patient population having JRA. In one embodiment, the effective TNFα inhibitor was previously identified as achieving a Ped ACR90 response in at least about 21 % of a patient population having JRA. In one embodiment, the effective TNFα inhibitor was previously identified as achieving a Ped ACR90 response in at least about 22% of a patient population having JRA. In one embodiment, the effective TNFα inhibitor was previously identified as achieving a Ped ACR90 response in at least about 30% of a patient population having JRA. In one embodiment, the effective TNFα inhibitor was previously identified as achieving a Ped ACR90 response in at least about 42% of a patient population having JRA.

In one embodiment, the patient population was also administered methotrexate.

It should be noted that the Examples provided herein represent different methods of determining the efficacy of a TNFα inhibitor, such as a human TNFα antibody, or antigen-binding portion thereof. As such, data and results described in the Examples section which shows efficacy of a TNFα inhibitor, *e.g*., ability to prevent flare-ups in JRA, are included in the methods of determining efficacy of the invention.

Time points for determining efficacy will be understood by those of skill in the art to depend on the type of efficacy being determined, *e.g*., prevention of flare-ups. In one embodiment, measurements in scores, *e.g*., an PedACR 30, 50, 70, and/or 90 response, may be measured against a subject's baseline score. Generally, a baseline refers to a measurement or score of a patient before treatment, *i.e*. week 0. Other time points may also be included as a starting point in determining efficacy, however. For example, in determining the efficacy of a TNFα inhibitor for preventing flare-ups in a patient population having JRA, a determination of the percentage of the patient population who have not had a flare up, may be determined based on a time point from when a flare up was last recorded

Patient populations described in the methods of the invention are generally selected based on common characteristics, such as, but not limited to, subjects diagnosed with JRA who are being treated with a TNFα inhibitor. Such a patient population would be appropriate for determining the efficacy of the TNFα inhibitor for the treatment of JRA, including the prevention of flare-ups, in the given patient population. In one embodiment, the patient population is a population of children having JRA.

In one embodiment, the methods of the invention for determining whether a TNFα inhibitor is an effective TNFα inhibitor, include determining changes, improvements, measurements, etc., in JRA using appropriate indices known in the art, *e.g*., PedACR responses, flare-up numbers and/or time inbetween, from a patient population who has already been administered the TNFα inhibitor. Such a patient population may be pre-selected according to common characteristics, *e.g*., JRA, loss of response to infliximab, and may have already been given the TNFα inhibitor. Administration of the TNFα inhibitor may or may not be performed by the same person of ordinary skill who is determining the efficacy of the TNFα inhibitor in accordance with the teachings of the specificaiton.

In one embodiment, the methods of the invention comprise administering the TNFα inhibitor to the subjects of a patient population and determining the efficacy of the TNFα inhibitor by determining changes, improvements, measurements, etc., using JRA indices known in the art, in the patient population in comparison to the Examples set forth below. For example, in one embodiment the invention includes a method for determining efficacy of a TNFα inhibitor for the treatment of JRA comprising administering the TNFα inhibitor to a preselected patient population having JRA; and determining the effectiveness of the TNFα inhibitor by determining a PedACR response from the baseline of the patient population, wherein a ACR90 response achieved in at least about 15% of the patient population indicates that the TNFα inhibitor is effective for the treatment of JRA.

Methods of the invention relating to determining efficacy, *i.e*., determining whether a TNFα inhibitor is an effective TNFα inhibitor, may also be applied to specific patient populations within the overall patient population who together have specific, common characteristics, *i.e*., a subpopulation.

In addition, while the above methods are described in terms of patient populations, methods of efficacy described herein may also be applied to individual subjects. For example, a method for determining efficacy may comprise determining whether a subject who has JRA, and who is on a dosage regimen comprising a human TNFα antibody, is able to prevent flare-ups if the human TNFα antibody is an effective human TNFα antibody. In one embodiment, if the subject is able to prevent flare-ups for at least about 14 weeks, then the human TNFα antibody is effective at preventing flare-ups associated with JRA.

The Examples and discoveries described herein are representative of a TNFα inhibitor, *i.e*., adalimumab, which is effective for treating JRA, including preventing flare-ups. As such, the studies and results described in the Examples section herein may be used as a guideline for determining the efficacy of a TNFα inhibitor, *i.e*., whether a TNFα inhibitor is an effective TNFα inhibitor for the treatment of JRA. In one embodiment, methods of determining efficacy described herein may be used to determine whether a TNFα inhibitor is bioequivalent to another TNFα inhibitor.

In one embodiment, the article of manufacture of the invention comprises instructions regarding how to determine the efficacy of the TNF inhibitor for the treatment of JRA.

The present invention is further illustrated by the following Examples which should not be construed as limiting in any way.

### Example 1: 48-Week Data from the Study of Adalimumab in Children with Juvenile Idiopathic Arthritis (JIA)

From previously reported, preliminary, open-label data from 171 patients with JIA, adalimumab (Ada) was shown to improve signs and symptoms after 16 weeks of therapy (Fed ACR30 =83%). Approximately 2/3rds of responders achieved at least a Ped ACR70 response. The purpose of this analysis was to study the efficacy and safety of Ada in patients with JIA who responded to Ada during the initial, open-label study.

This was a multi-center, Phase III, randomized, double-blind, placebo-controlled withdrawal study of patients who had achieved a Ped PCR30 response during the open-label, lead-in phase (study design described in Figure 1). Eligible patients were 4-17 years of age and had polyarticular-course JIA, with a minimum of 5 swollen joints and 3 joints with limitation of motion (LOM). During a succeeding, double-blind period, patients were randomized to either active treatment (24 mg Ada/M² BSA sc cow) or placebo, and were followed for an additional 32 weeks to determine if their disease had flared (primary endpoint). The disese flare criteria were 1) a worsening of >30% from baseline in 3 of the 6 core criteria for the Ped ACR, 2) no fewer than 2 active joints, and 3) improvement of >30% in no more than 1 of the 6 criteria. Patients who flared were allowed to begin receiving open-label Ada therapy. Randomizaton was stratified by the presence or absence of concomitant methotrexate (MTX) treatment. Efficacy and safety assessments were collected at regular intervals.

At the end of the 16-week, open-label lead-in phase, the ACR response rates were 83% for ACR20, 74% for ACR50, and 52% for ACR70. A total of 133 patients entered the double-blind phase: 77% were female, mean age was 11.2 years, and 65% were taking concomitant MTX. Overall, 96% of patients who entered the double-blind phase completed 32 weeks of therapy, and 4% discontinued (1 protocol violation, 1 withdrew consent, 3 other). Compared with placebo, significantly fewer patients receiving Ada had disease flares, regardless of concomitant MTX use. This was seen both in patients *not* receiving MTX (43.3% vs. 71.4%, p=0.031, primary endpoint), and in patients receiving MTX (36.8% vs. 64.9%, p=0.015). In patients receiving Ada, ACR30/50/70 responses at Week 48 (end of double-blind period) were statistically significantly greater than responses in placebo patients.

**Table 1.**

| Pediatric ACR Responders at Week 48 | | |
|---|---|---|
| | Ada | Placebo |
| | (n=68) | (n=65) |
| ACR30 | 60%* | 35% |
| ACR50 | 59%* | 35% |
| ACR70 | 56%* | 28% |

| | | |
|---|---|---|
| * p<0.01 for adalimumab vs placebo. | | |

Even with stringent flare criteria, patients who flared still had very good ACR response rates at the time of flare; 73% still had an ACR30, 61% had an ACR50, and 24% hand an ACR70. These patients were considered non-responders for the Week-48 analysis. Moreover, patients who did not flare maintained or imporoved their ACR response rates from Week 16 to Week 48. ACR30 scores for Ada patients who did not flare were 100% and 98% at Weeks 16 and 48, respectively. Comparable numbers for ACR50, ACR70 and ACR90 were 90 and 97; 65 and 90; and 39 and 65.

The most common AEs were infections (predominantly mild upper respiratory infections). Four Ada patients and two placebo patients experienced SAEs during the blinded phase. No TB or opportunistic infections were reported.

In conclusion, either alone or with MTX, Ada provided rapid and substantial improvement in signs and symptoms of disease in children with active JIA, and that improvement was sustained for up to 1 year. Despite a stringent definition of disease flare, Ada patients had a significantly lower rate of flares (vs. placebo) and maintained substantial ACR responses even when flares occurred. Ada was generally well-tolerated in children with JIA.

### Example 2: Long-Term Efficacy and Safety of Adalimumab in Children with Juvenile Rheumatoid Arthritis (JRA): 48-Week Results

The below study expands on the study described in Example 1.

From previously reported, preliminary, open-label data from patients with JRA, adalimumab (ADA), 24 mg/M² BSA administered subcutaneously every two weeks, was shown to improve signs and symptoms after 16 weeks of therapy (Ped ACR70 =50%).

The below study expands on the study described in Example 1. In the below study, the ACR responses of patients with JRA were observed in a subsequent 32-week, double-blind study. The efficacy objectives of the present study were to compare disease flare in adalimumab (ada)-treated patients to placebo-treated patients in the non-MTX stratum during the double-blind phase and to determine time to onset of flare in adalimumab-treated & placebo-treated patients in non-MTX & MTX strata. An additional objective were to determine continued clinical benefit at 30%, 50%, and 70% response levels after repeated subcutaneous (sc) administration of adalimumab. In addition to the efficacy objective, the safety objectives were to evaluate the long-term safety profile of adalimumab treatment, as a monotherapy and in combination with MTX, in JRA patients. Figure 1 depicts the study design.

Eligible patients were 4-17 years of age and had polyarticular JRA, with a minimum of 5 swollen joints and 3 joints with limitation of motion (LOM). Other inclusion criteria included prior adequate trial of NSAIDs and stable MTX dosage. Ineligible patients included those in functional class IV by ACR criteria who had joint surgery within 2 months of screening, ongoing chronic or active infection, or significant active concomitant illness. Baseline patient characteristics are shown in Table 1 below.

**Table 1. Baseline Patient Characteristics**

| | MTX (n=85) | Non-MTx (n=86) |
|---|---|---|
| Age (years) | 11.4 | 11.1 |
| Female (%) | 80 | 78 |
| Positive RF (%) | 22 | 21 |
| JRA Duration (yrs) | 4.0 | 3.6 |
| Physician Global | 58.2 | 59.7 |
| Parent Global | 42.9 | 53.7 |
| # active joints | 15.0 | 19.4 |
| # LOM joints | 12.8 | 14.3 |
| CHAQ | 0.9 | 1.2 |
| CRP | 2.4 | 2.9 |

During a succeeding, double-blind period, patients were randomized to either active treatment (24 mg Ada/M² BSA sc eow) or placebo, and were followed for an additional 32 weeks to determine if their disease had flared (primary endpoint). Patient disposition is depicted in Figure 2. The disease flare criteria included 1) a worsening of >30% in 3 of the 6 core criteria (from baseline of double-blind period), 2) no fewer than two active joints, and 3) improvement of 30% in no more than one criteria. If global assessment was used for flare criteria, >30% increase in disease activity on a scale of 0-100. Patients who flared discontinued from double-blind treatment and became eligible for open-label adalimumab therapy.

Patients who flared were allowed to discontinue double-blind treatment and begin receiving open-label ADA therapy. Randomizaton was stratified by the presence or absence of concomitant methotrexate (MTX) treatment.

Efficacy was recorded by ACR pediatric 30: at least 30% improvement from baseline in 3 of the following 6 core set variables, with no more than one of the remaining variables worsening by >30%. Efficacy and safety assessments, including physician global assessment of disease activity, parent/patient assessment of overall well-being, functional ability (disability index of the Childhood Health Assessment Questionnaire - CHAQ), number of joints with active arthritis, number of joints with limited range of motion, and lab measure of acute inflammation (CRP) were collected at regular intervals. Core set variables: 1) Physician global assessment of disease activity; 2) Parent/patient assessment of overall well-being; 3) Functional ability (disability index of the CHAQ); 4) Number of joints with active arthritis; 5) Number of joints with limited range of motion; and 6) C-reactive protein (CRP).

Tables 2 and 3 report the baseline demographics and disease activity beginning the Double-Blind Phase. The average age of the patient was 11 years, approximately 80% female, with most patients having JRA 3-4 years.

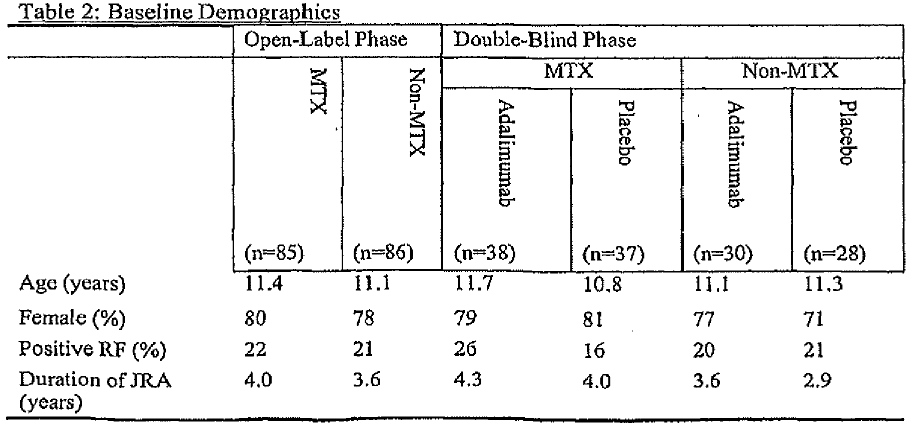

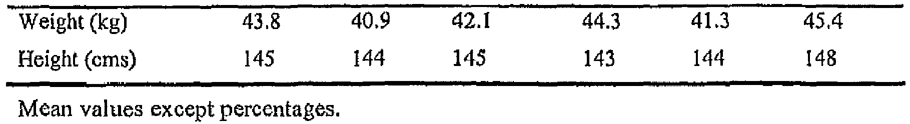

**Table 3 : Baseline Disease Activity for The Double-Blind Phase**

| | MTX | | Non-MTX | |
|---|---|---|---|---|
| | Adalimumab | Placebo | Adalimumab | Placebo |
| | (n=38) | (n=37) | (n=30) | (n=28) |
| | | | | |
| | | | | |
| SJC | 13.5 | 13.6 | 15.4 | 17.5 |
| (Range) | (3-31) | (5-33) | (5-42) | (6-44) |
| TJC | 11.5 | 8.8 | 13.2 | 14.7 |
| (Range) | (0-48) | (0-38) | (0-47) | (0-65) |
| Active Joints | 15.4 | 15.2 | 18.8 | 20.0 |
| (Range) | (5-35) | (5-33) | (5-49) | (7-50) |
| POM | 8.9 | 7.3 | 11.3 | 10.5 |
| (Range) | (0-29) | (0-27) | (0-40) | (0-70) |
| CHAQ | 1.0 | 0.9 | 1.1 | 1.3 |
| (Range) | (0-2) | (0-2) | (0-3) | (0-3) |
| CRP | 2.4 | 2.6 | 2.2 | 2.6 |
| (Range) | (0-11) | (0-18) | (0-24) | (0-16) |

**Table 4: Patient Disposition**

| | | Open-Label Phase | | Double-Blind Phase | | | |
|---|---|---|---|---|---|---|---|
| | | | | MTX | | Non-MTX | |
| | | MTX n (%) | Non-MTX n (%) | Adalimumab n (%) | Placebo n (%) | Adalimumab n (%) | Placebo n (%) |
| | | (n=85) | (n=86) | (n=38) | (n=37) | (n=30) | (n=28) |
| Completed | | 83 (98) | 77 (90) | 35 (92) | 36 (97) | 29 (97) | 28 (100) |
| Discontinued | | 2 (2) | 9 (11) | 3 (8) | 1 (3) | 1 (3) | 0 (0) |
| Reason: | | | | | | | |
| | *Protocol* Violation | 0 (0) | 0 (0) | 0 (0) | 0 (0) | 1 (3) | 0 (0) |
| | Withdrew Consent | 0 (0) | 0 (0) | 0 (0) | 1 (3) | 0 (0) | 0 (0) |
| | AE | 1 (1) | 2 (2) | 0 (0) | 0 (0) | 0 (0) | 0 (0) |
| | Lack of efficacy | 0 (0) | 6 (7) | 0 (0) | 0 (0) | 0 (0) | 0 (0) |
| | Other | 1 (1) | 1 (1) | 3 (8) | 0 (0) | 0 (0) | 0 (0) |

At the end of the 16-week, open-label lead-in phase, the ACR response rates in the ADA + MTX group were 93% for ACR30, 88% for ACR50, 65% for ACR70, and 20% for ACR90. In the group of patients who received ADA alone (non-MTX), the ACR response rates were 73% for ACR30, 59% for ACR50, 39% for ACR70, and 22% for ACR90. The ACR response rates at the end fo the 16-week open-label lead-in phase are shown in Table 2.

**Table 2: ACR Response Rates by MTX Use at Week 16: End of Open Label Phase**

| | MTX | Non-MTX | Overall |
|---|---|---|---|
| ACR 30 | 93 | 73 | 83 |
| ACR 50 | 88 | 59 | 74 |
| ACR 70 | 65 | 39 | 52 |
| ACR 90 | 20 | 22 | 21 |

| | | | |
|---|---|---|---|
| Numbers represent percent of patients. Patients who dropped out of the study or had missing data were considered non-responders | | | |

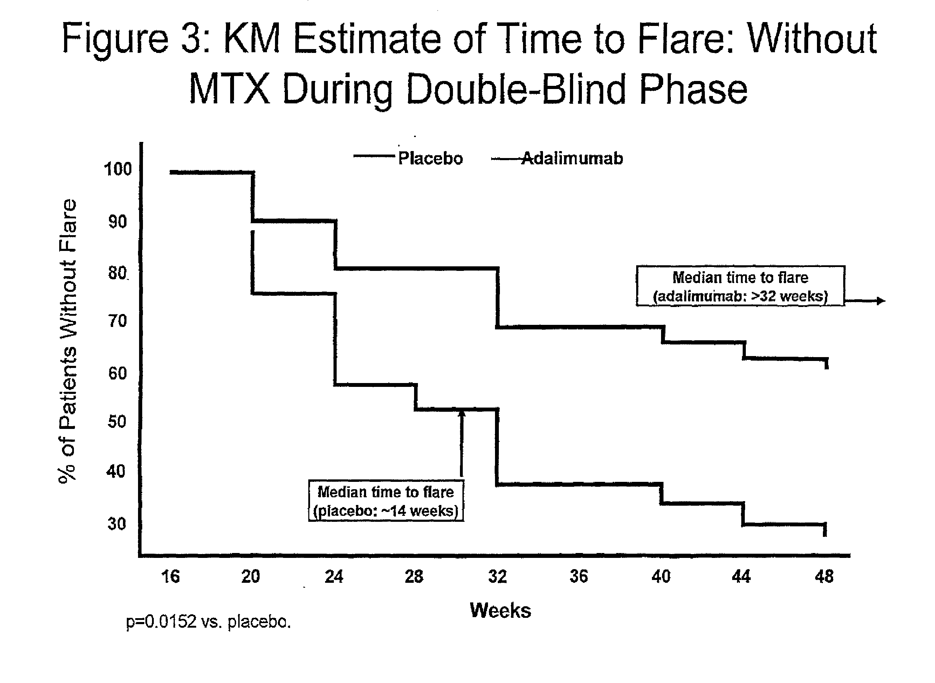

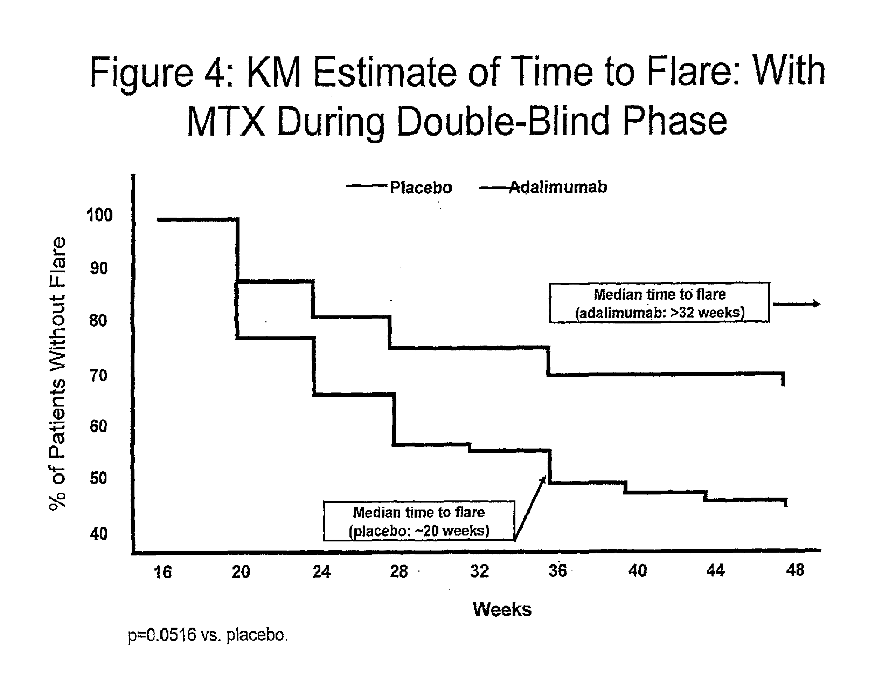

The KM estimate of time to flare without MTX during the double blind phase is depicted in Figure 3, and the KM estimate of time to flare with MTX is depicted in Figure 4.

By week 48, significantly fewer patients receiving either ADA alone or ADA + MTX had flared compared with placebo. Of the patients who received placebo + MTX or ada + mtx, 65 percent of the patients receiving placebo flared while only 37% of the patients receiving adalimumab flared. Similarly, of the patients in the study without MTX, 71% of the placebo patients flared versus only 43% receiving adalimumab.

The ACR response rates by MTX use at week 48 of non-responders (patients who flared) are shown in Table 3. The table reveals a reduction in ACR for those patients switched to the placebo in the double blind phase.

**Table 3: ACR Response Rates by MTX Use at Week 48: End of Open Label Phase**

| | MTX | | Non-MTX | |
|---|---|---|---|---|
| | Adalimumab | Placebo | Adalimumab | Placebo |
| ACR30 | 63 | 38 | 57 | 32 |
| ACR 50 | 63 | 38 | 53 | 32 |
| ACR 70 | 63 | 27 | 47 | 29 |
| ACR 90 | 42 | 27 | 30 | 18 |

| | | | | |
|---|---|---|---|---|
| *p<0.05, †p<0.01 vs placebo. Numbers represent percent of patients. Patients who flared were considered non-responders. | | | | |

The ACR response rates at the time of flare for all of the patients who flared were as follows: 73% had an ACR30 response, 61% had an ACR50 response, 24% had an ACR70 response, and 4% had an ACR90 response.

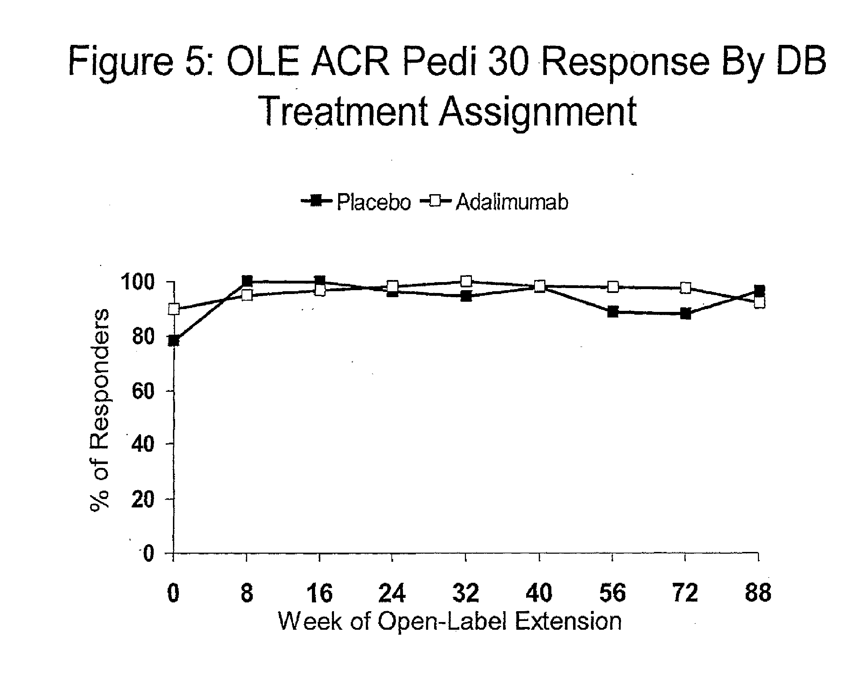

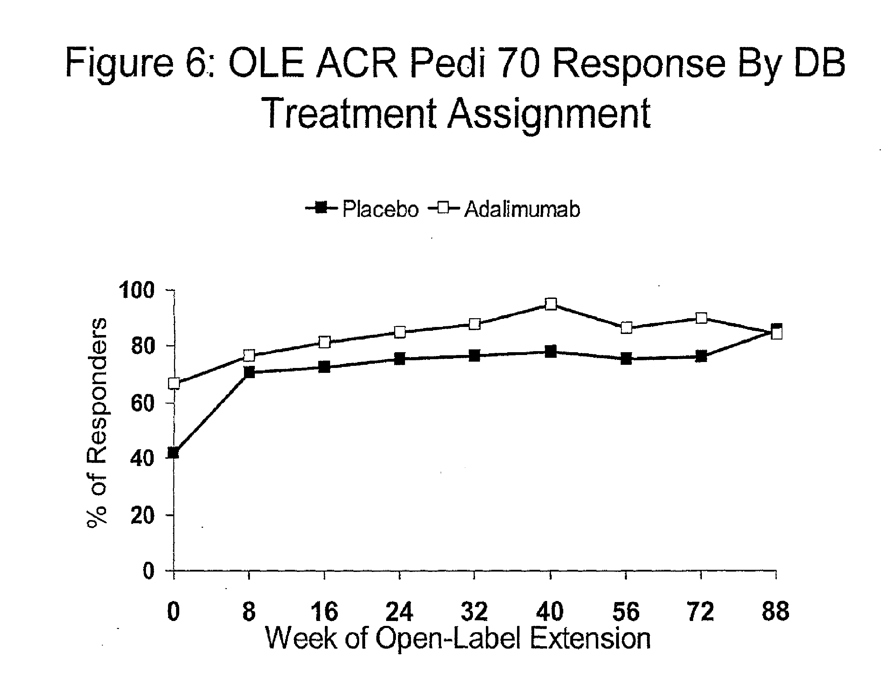

The JRA open label extension (OLE) is depicted in Figure 1. The OLE ACR Pedi 30 and 70 responses by DB treatment assignment are depicted in Figure 5 and Figure 6, respectively.

The most common AEs were infections, as shown below in Table 4. No TB or opportunistic infections were reported. The treatment-emergent serious adverse events which occurred in the open-label phase included leukopenia, neutropenia and JRA in patients who received ADA + MTX, and herpes genitalis, JRA and pneumonia in patients who received ADA alone. Treatment -emergent serious adverse events between the open-label and double-blind phases are shown in Table 5.

**Table 4. Overview of Treatment-Emgergent Adverse Events**

| | Open-Label | | Double-Blind | | | |
|---|---|---|---|---|---|---|
| | | | MTX | | Non-MTX | |
| Adverse Event | ADA + | ADA | ADA | PBO | ADA | **PBO** |
| | MTX n(%) | n(%) | n(%) | n(%) | n(%) | n(%) |
| | (n=85) | (n=86) | (n=38) | (n=37) | (n=30) | (n=28) |
| Injection site reactions | 35 (41) | 37 (43) | 12 (37) | 9 (24) | 11 (37) | 4 (14) |
| Infections | 37 (44) | 39 (45) | 22 (58) | 19 (51) | 19 (63) | 11 (39) |
| Serious infections | 0 (0) | 2 (2) | 1 (3) | 0 (0) | 1 (3) | 0 (0) |

**Table 5. Treatment-Emergent Serious Adverse Events**

| Open-Label | | Double-Blind | | | |
|---|---|---|---|---|---|
| | | MTX | | Non-MTX | |
| ADA + MTX | ADA | ADA | PBO | ADA | PBO |
| Leukopenia | Herpes Genitalis | Appendicitis | Grastro-duodenitis | Urinary tract infection | |
| neutropenia | JRA (3) | abdominal pain | retinal detachment | | |
| JRA | pneumonia | injury | | | |

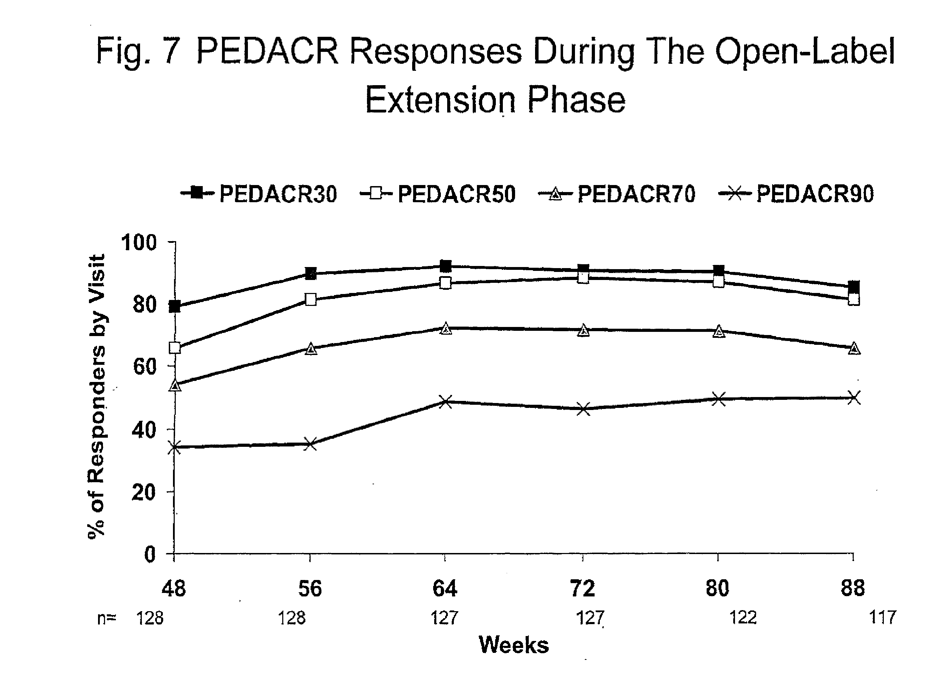

In conclusion, treatment with adalimumab, either alone or in combination with MTX, provided substantial clinical improvement in children with active JRA. Responses were maintained through 88 weeks of open-label treatment as shown in Figure 7. Open-label adalimumab treatment resulted in a substantial increase in the percentage of PedACR30, 50 and 70 responders in those subjects randomized to placebo during the DB phase. Overall, adalimumab was shown to be generally safe and well-tolerated in children with JRA.

### EQUIVALENTS

Those skilled in the art will recognize, or be able to ascertain using no more than routine experimentation, many equivalents to the specific embodiments of the invention described herein. Such equivalents are intended to be encompassed by the following claims. The contents of all references, patents and published patent applications cited throughout this application are incorporated herein by reference.

In addition, the present invention further relates to the following items:
1. Use of a TNFα antibody, or antigen-binding portion thereof, in the manufacture of a medicament for preventing a flare-up associated with juvenile rheumatoid arthritis (JRA).
2. The use of item 1, wherein the TNFα antibody, or antigen-binding portion thereof, is administered with methotrexate.
3. The use of item 2, wherein the flare-up is prevented for at least 20 weeks.
4. The use of item 1, wherein the TNFα antibody, or antigen-binding portion thereof, is administered without methotrexate.
5. The use of item 2, wherein the flare-up is prevented for at least 14 weeks.
6. The use of any one of items 1-5, wherein the TNFα antibody, or antigen-binding portion thereof, is administered at a dose of 24 mg/M² BSA.
7. The use of any one of items 1-5, wherein the TNFα antibody, or antigen-binding portion thereof, is administered subcutaneously.
8. The use of any one of items 1-5, wherein the TNFα antibody, or antigen-binding portion thereof, is administered on a biweekly dosing regimen.
9. A method for determining the efficacy of a TNFα antibody, or antigen-binding portion thereof, for the prevention of a flare-up associated with JRA in a subject, comprising
   determining a median time to flare of a patient population having JRA and who was administered the TNFα antibody, or antigen-binding portion thereof,
   wherein a median time to flare of at least about 14 weeks indicates that the TNFα antibody, or antigen-binding portion thereof, is an effective TNFα antibody, or antigen-binding portion thereof, for the prevention of flare associated with JRA.
10. The method of item 9, further comprising administering the effective TNFα antibody, or antigen-binding portion thereof, to a subject to prevent a flare-up associated with JRA.
11. The method of item 9 or 10, wherein a flare-up occurrence rate of about 40% or less indicates that the TNFα antibody, or antigen-binding portion thereof, is an effective TNFα antibody, or antigen-binding portion thereof, for the prevention of the flare-up associated with JRA.
12. The method of item 9 or 10, wherein a flare-up occurrence rate of about 37% or less indicates that the TNFα antibody, or antigen-binding portion thereof, is an effective TNFα antibody, or antigen-binding portion thereof, for the prevention of the flare-up associated with JRA.
13. A method of preventing a flare up in a subject having JRA comprising administering an effective TNFα antibody, or antigen-binding portion thereof, to a subject such that flare-up is prevented, wherein the effective TNFα antibody, or antigen-binding portion thereof, was previously identified as preventing a flare-up for at least about 14 weeks in a patient population having JRA.
14. A method for determining the efficacy of a TNFα antibody, or antigen-binding portion thereof, for the prevention of a flare-up associated with JRA in a subject, comprising
   determining a flare-up occurrence rate of a patient population having JRA and who was administered the TNFα antibody, or antigen-binding portion thereof,
   wherein a flare-up occurrence rate of about 43% or less indicates that the TNFα antibody, or antigen-binding portion thereof, is an effective TNFα antibody, or antigen-binding portion thereof, for the prevention of the flare-up associated with JRA.
15. The method of item 14, further comprising administering the effective TNFα antibody, or antigen-binding portion thereof, to a subject for the prevention of a flare-up associated with JRA.
16. Use of a TNFα antibody, or antigen-binding portion thereof, in the manufacture of a medicament for preventing a flare up in a subject having JRA comprising administering an effective TNFα antibody, or antigen-binding portion thereof, to a subject such that the flare-up is prevented, wherein the effective TNFα antibody, or antigen-binding portion thereof, was previously identified as preventing a flare-up in about 43% or less of a patient population having JRA.
17. The method or use of any one of items 14-16, wherein a flare-up occurrence rate of about 40% or less indicates that the TNFα antibody, or antigen-binding portion thereof, is an effective TNFα antibody, or antigen-binding portion thereof, for the prevention of the flare-up associated with JRA.
18. The method or use of any one of items 14-16, wherein a flare-up occurrence rate of about 37% or less indicates that the TNFα antibody, or antigen-binding portion thereof, is an effective TNFα antibody, or antigen-binding portion thereof, for the prevention of the flare-up associated with JRA.
19. The method or use of any one of items 1-18, wherein the patient population was also administered methotrexate.
20. The method or use of any one of items 1-18, wherein the patient population was administered the TNFα antibody, or antigen-binding portion thereof, without methotrexate.
21. A method for determining the efficacy of a TNFα inhibitor for the treatment of juvenile rheumatoid arthritis (JRA) in a subject comprising
   determining a Pediatric (Ped) ACR90 response of a patient population having JRA and who was administered the TNFα inhibitor,
   wherein a Ped ACR90 response achieved in at least about 15% of the patient population indicates that the TNFα inhibitor is an effective TNFα inhibitor for treating JRA.
22. The method of item 21, further comprising administering the effective TNFα inhibitor to a subject to treat JRA.
23. The method of item 22, wherein the TNFα inhibitor is administered either with or without methotrexate.
24. The method of any one of items 21-23, wherein a Ped ACR90 response achieved in at least about 20% of the patient population indicates that the TNFα inhibitor is an effective TNFα inhibitor for treating JRA.
25. The method of any one of items 21-23, wherein a Ped ACR90 response achieved in at least about 30% of the patient population indicates that the TNFα inhibitor is an effective TNFα inhibitor for treating JRA.
26. The method of items 21-23, wherein a Ped ACR90 response achieved in at least about 40% of the patient population indicates that the TNFα inhibitor is an effective TNFα inhibitor for treating JRA.
27. Use of a TNFα antibody, or antigen-binding portion thereof, in the manufacture of a medicament for treating JRA in a subject comprising administering an effective TNFα inhibitor to the subject such that JRA is treated, wherein the effective TNFα inhibitor was previously identified as achieving a PedACR90 response in at least about 15 % of a patient population having JRA.
28. The use of item27, further comprising administering methotrexate to the subject.
29. The use ofitem 27 or 28, wherein the effective TNFα inhibitor was previously identified as achieving a Ped ACR90 response in at least about 20% of a patient population having JRA.
30. The use of item 27 or 28, wherein the effective TNFα inhibitor was previously identified as achieving a Ped ACR90 response in at least about 30% of a patient population having JRA.
31. The use of item 27 or 28, wherein the effective TNFα inhibitor was previously identified as achieving a Ped ACR90 response in at least about 40% of a patient population having JRA.
32. The use of item27 or 28, wherein the effective TNFα inhibitor was previously identified as achieving a Ped ACR90 response in at least about 42% of a patient population having JRA.
33. The method or use of anyone of items21-32, wherein the TNFα inhibitor is selected from the group consisting of a TNFα antibody, or an antigen-binding portion thereof, a TNFα fusion protein, or a recombinant TNFα binding protein.
34. The method or use of item 33, wherein the TNFα fusion protein is etanercept.
35. The method or use of any one of items 1-20 and 33, wherein the TNFα antibody, or antigen-binding portion thereof, is infliximab or golimumab.
36. The method or use of any one of items 1-20 and 33, wherein the TNFα antibody, or antigen-binding portion thereof, is selected from the group consisting of a human antibody, a chimeric antibody, a multivalent antibody, and a humanized antibody.
37. The method or use of item 36, wherein the TNFα antibody, or antigen-binding portion thereof, is an isolated human antibody that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.
38. The method or use of item 36, wherein the TNFα antibody, or antigen-binding portion thereof, is an isolated antibody, or antigen-binding portion thereof, with the following characteristics:
   a) dissociates from TNFα with a K_{off} rate constant of 1 x 10⁻³ s⁻¹ or less, as determined by surface plasmon resonance;
   b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
   c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9,10,11 and/or 12.
39. The method or use of item 36, wherein the TNFα antibody, or antigen-binding portion thereof, is an isolated human antibody, or an antigen binding portion thereof, with a light chain variable region (LCVR) comprising the amino acid sequence of SEQ ID NO: 1 and a heavy chain variable region (HCVR) comprising the amino acid sequence of SEQ ID NO: 2.
40. The method or use of item 36, wherein the TNFα antibody, or antigen-binding portion thereof, is adalimumab.
41. The method or use of any one of items 36-40, wherein the TNFα antibody, or antigen-binding portion thereof, is administered at a dose of 24 mg/M² BSA.
42. The method or use of any one of items 36-40, wherein the TNFα antibody, or antigen-binding portion thereof, is administered subcutaneously.
43. The method or use of any one of items 36-40, wherein the TNFα antibody, or antigen-binding portion thereof, is administered on a biweekly dosing regimen.
44. The method or use of any one of items 1-43, wherein JRA is polyarticular JRA.
45. An article of manufacture comprising
   a) a packaging material;
   b) a TNFα antibody, or antigen-binding portion thereof; and
   c) a label or package insert indicating that in studies of the TNFα antibody, or antigen-binding portion thereof, for the treatment of juvenile rheumatoid arthritis (JRA) the most common adverse events (AEs) were infections.
46. An article of manufacture comprising
   a) a packaging material;
   b) a TNFα antibody, or antigen-binding portion thereof; and
   c) a label or package insert indicating that in studies of the TNFα antibody, or antigen-binding portion thereof, the TNFα antibody was shown to prevent flare-ups in patients having JRA.
47. The article of item 45 or 46, wherein the TNFα antibody, or antigen-binding portion thereof, is selected from the group consisting of adalimumab, infliximab, and golimumab.
48. The article of item 46 or 46, wherein the TNFα antibody, or antigen-binding portion thereof, is an isolated human antibody that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of I x 10⁻⁷ M or less.

## Claims

1. An isolated human anti-TNFα antibody, or antigen binding portion thereof, for use in maintaining a Paediatric ACR30 response in a human subject with juvenile rheumatoid arthritis for up to 1 year,
wherein the anti-TNFα antibody, or an antigen-binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

2. An isolated human anti-TNFα antibody, or antigen binding portion thereof, for use in treating juvenile rheumatoid arthritis in a human subject, wherein the anti-TNFa antibody, or antigen binding portion thereof, is administered to a human subject for 16 weeks,
wherein if the human subject achieves a Paediatric ACR30 response after the 16 weeks, the anti-TNFa antibody, or antigen binding portion thereof, is administered to the human subject for an additional 32 weeks; and
wherein the anti-TNFα antibody, or an antigen-binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

3. An isolated human anti-TNFα antibody, or antigen binding portion thereof, for use in treating juvenile rheumatoid arthritis (JRA) in a patient population having JRA, wherein the anti-TNFα antibody, or antigen binding portion thereof, achieves an ACR30 response in at least about 60% of the patient population after 48 weeks of therapy,
wherein the anti-TNFα antibody, or an antigen-binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

4. An isolated human anti-TNFα antibody, or antigen binding portion thereof, for use in treating juvenile rheumatoid arthritis(JRA) in a patient population having JRA, wherein the anti-TNFα antibody, or antigen binding portion thereof, prevents a flare-up in at least about 43% of the patient population after 48 weeks of therapy,
wherein the anti-TNFα antibody, or an antigen-binding portion thereof, comprises a light chain variable region (LCVR) having a CDR1 domain comprising the amino acid sequence of SEQ ID NO: 7, a CDR2 domain comprising the amino acid sequence of SEQ ID NO: 5, and a CDR3 domain comprising the amino acid sequence of SEQ ID NO: 3; and a heavy chain variable region (HCVR) having a CDR1 domain comprising the amino acid sequence of SEQ ID NO: 8, a CDR2 domain comprising the amino acid sequence of SEQ ID NO: 6, and a CDR3 domain comprising the amino acid sequence of SEQ ID NO: 4.

5. The isolated human anti-TNFα antibody, or antigen binding portion thereof of any one of claims 1-4, wherein the light chain variable region (LCVR) comprises the amino acid sequence of SEQ ID NO: 1 and the heavy chain variable region (HCVR) comprises the amino acid sequence of SEQ ID NO: 2.

6. The isolated human anti-TNFα antibody, or antigen binding portion thereof of any one of claims 1-4, wherein the antibody is adalimumab.

7. The isolated human anti-TNFa antibody, or antigen binding portion thereof of any one of claims 1, 2, 5, or 6, wherein the anti-TNFα antibody, or antigen binding portion thereof is administered to the subject on a biweekly dosing regimen.

8. The isolated human anti-TNFa antibody, or antigen binding portion thereof of any one of claims 1, 2, or 5-7, wherein the anti-TNFα antibody, or antigen binding portion thereof is administered to the subject subcutaneously.

9. The isolated human anti-TNFα antibody, or antigen binding portion thereof of any one of claims 1, 2 or 5-8, wherein the anti-TNFα antibody, or antigen binding portion thereof is administered to the subject at a dose of 24 mg/M² BSA.

10. The isolated human anti-TNFa antibody, or antigen binding portion thereof of any one of claims 3-6, wherein the anti-TNFα antibody, or antigen binding portion thereof is administered to the patient population in a biweekly dosing regimen.

11. The isolated human anti-TNFa antibody, or antigen binding portion thereof of any one of claims 3-6 or 10, wherein the anti-TNFα antibody, or antigen binding portion thereof is administered to the patient population subcutaneously.

12. The isolated human anti-TNFα antibody, or antigen binding portion thereof of any one of claims 3-6, 10, or 11, wherein the anti-TNFa antibody, or antigen binding portion thereof is administered to the patient population at a dose of 24 mg/M² BSA.
